# EUROPÄISCHE PATENTSCHRIFT

(11) **EP 2 133 420 B1**
(45) Veröffentlichungstag und Bekanntmachung des Hinweises auf die Patenterteilung: **23.04.2014**
(21) Anmeldenummer: 09162040.1
(22) Anmeldetag: 05.06.2009
(51) Int. Cl.: C12N 9/88, C12P 13/04, C12P 13/06, C12P 13/08, C12P 13/22, C12N 15/60

(54) **Verfahren zur Herstellung von L-Aminosäuren unter Verwendung von verbesserten Stämmen der Familie Enterobacteriaceae**
Method for manufacturing L-amino acids using improved strains of the enterobacteriaceae family
Procédé de fabrication d'aminoacides L en utilisant des troncs améliorés de la famille des enterobacteriaceae

(30) Priorität: 09.06.2008 DE 102008002309
(43) Veröffentlichungstag der Anmeldung: 16.12.2009
(73) Patentinhaber: Evonik Degussa GmbH, 45128 Essen (DE)
(72) Erfinder: Rieping, Mechthild Dr., 33619 Bielefeld (DE); Jerrentrup, Silke, 33607 Bielefeld (DE); Wiegräbe, Iris, 33613 Bielefeld (DE); Kreutzer, Caroline, 33813 Oerlinghausen (DE)

(56) Entgegenhaltungen:
- WO-A-2006/015669
- WO-A-2008/072640
- GUILLOTON M B ET AL: "Carbonic anhydrase in Escherichia coli. A product of the cyn operon." THE JOURNAL OF BIOLOGICAL CHEMISTRY 25 FEB 1992, Bd. 267, Nr. 6, 25. Februar 1992 (1992-02-25), Seiten 3731-3734, XP002542170 ISSN: 0021-9258
- HILTONEN T ET AL: "Intracellular beta-carbonic anhydrase of the unicellular green alga Coccomyxa. Cloning of the cdna and characterization of the functional enzyme overexpressed in Escherichia coli." PLANT PHYSIOLOGY AUG 1998, Bd. 117, Nr. 4, August 1998 (1998-08), Seiten 1341-1349, XP002542171 ISSN: 0032-0889
- KUSIAN BERNHARD ET AL: "Carbonic anhydrase is essential for growth of Ralstonia eutropha at ambient CO(2) concentrations." JOURNAL OF BACTERIOLOGY SEP 2002, Bd. 184, Nr. 18, September 2002 (2002-09), Seiten 5018-5026, XP002542172 ISSN: 0021-9193

### Sequenzprotokoll

<110> Evonik Degussa GmbH
<120> Verfahren zur Herstellung von L-Aminosäuren unter Verwendung von verbesserten Stämmen der Familie Enterobacteriaceae
<130> 200800181
<160> 18
<170> Patent In version 3.3
<210> 1
   <211> 663
   <212> DNA
   <213> Escherichia coli
<220>
   <221> CDS
   <222> (1)..(663)
   <223> can Kodierregion
<400> 1
   atg aaa gac ata gat aca ctc atc agc aac aat gca cta tgg tca aaa        48
   Met Lys Asp Ile Asp Thr Leu Ile Ser Asn Asn Ala Leu Trp Ser Lys
   1               5                   10                  15
   atg ctg gtg gaa gag gat ccc ggg ttt ttt gag aaa ctg gca caa gcg        96
   Met Leu Val Glu Glu Asp Pro Gly Phe Phe Glu Lys Leu Ala Gln Ala
               20                  25                  30
   caa aaa ccg cgc ttt cta tgg att gga tgt tcc gac agt cgc gtt cct       144
   Gln Lys Pro Arg Phe Leu Trp Ile Gly Cys Ser Asp Ser Arg Val Pro
           35                  40                  45
   gca gaa cgt tta acc ggt ctt gag ccg ggc gaa ctc ttt gtt cac cgt       192
   Ala Glu Arg Leu Thr Gly Leu Glu Pro Gly Glu Leu Phe Val His Arg
       50                  55                  60
   aat gtt gct aac ctg gtc att cac act gac ctg aac tgc ctt tcc gtg       240
   Asn Val Ala Asn Leu Val Ile His Thr Asp Leu Asn Cys Leu Ser Val
   65                  70                  75                  80
   gtt cag tat gca gtg gat gta ctc gaa gtt gaa cac att att atc tgt       288
   Val Gln Tyr Ala Val Asp Val Leu Glu Val Glu His Ile Ile Ile Cys
                   85                  90                  95
   ggc cac tac ggt tgc ggc ggc gta caa gcc gca gtt gaa aac ccg gaa       336
   Gly His Tyr Gly Cys Gly Gly Val Gln Ala Ala Val Glu Asn Pro Glu
               100                 105                 110
   ctg ggg ctt atc aac aac tgg ctg ctg cat atc cgc gat atc tgg ttc       384
   Leu Gly Leu Ile Asn Asn Trp Leu Leu His Ile Arg Asp Ile Trp Phe
           115                 120                 125
   aaa cat agc tca ttg ctc ggc gaa atg ccg caa gag cgc cgt ctg gat       432
   Lys His Ser Ser Leu Leu Gly Glu Met Pro Gln Glu Arg Arg Leu Asp
       130                 135                 140
   acc ttg tgt gaa ctg aac gtc atg gaa cag gtg tat aac ctg ggc cac       480
   Thr Leu Cys Glu Leu Asn Val Met Glu Gln Val Tyr Asn Leu Gly His
   145                 150                 155                 160
   tcc acc att atg caa tca gcg tgg aaa cgc ggg cag aaa gtt acc att       528
   Ser Thr Ile Met Gln Ser Ala Trp Lys Arg Gly Gln Lys Val Thr Ile
                   165                 170                 175
   cac ggc tgg gcc tac ggc att cac gac ggc ttg ctg cgt gat ctg gat       576
   His Gly Trp Ala Tyr Gly Ile His Asp Gly Leu Leu Arg Asp Leu Asp
               180                 185                 190
   gtt acc gcc acc aac cgc gaa acc ctt gag caa cgt tac cgt cac ggg       624
   Val Thr Ala Thr Asn Arg Glu Thr Leu Glu Gln Arg Tyr Arg His Gly
           195                 200                 205
   att tcc aac ctc aag ctg aaa cac gcc aac cac aaa taa                   663
   Ile Ser Asn Leu Lys Leu Lys His Ala Asn His Lys
       210                 215                 220
<210> 2
   <211> 220
   <212> PRT
   <213> Escherichia coli
<400> 2
   Met Lys Asp Ile Asp Thr Leu Ile Ser Asn Asn Ala Leu Trp Ser Lys
   1               5                   10                  15
   Met Leu Val Glu Glu Asp Pro Gly Phe Phe Glu Lys Leu Ala Gln Ala
               20                  25                  30
   Gln Lys Pro Arg Phe Leu Trp Ile Gly Cys Ser Asp Ser Arg Val Pro
           35                  40                  45
   Ala Glu Arg Leu Thr Gly Leu Glu Pro Gly Glu Leu Phe Val His Arg
       50                  55                  60
   Asn Val Ala Asn Leu Val Ile His Thr Asp Leu Asn Cys Leu Ser Val
   65                  70                  75                  80
   Val Gln Tyr Ala Val Asp Val Leu Glu Val Glu His Ile Ile Ile Cys
                   85                  90                  95
   Gly His Tyr Gly Cys Gly Gly Val Gln Ala Ala Val Glu Asn Pro Glu
               100                 105                 110
   Leu Gly Leu Ile Asn Asn Trp Leu Leu His Ile Arg Asp Ile Trp Phe
           115                 120                 125
   Lys His Ser Ser Leu Leu Gly Glu Met Pro Gln Glu Arg Arg Leu Asp
       130                 135                 140
   Thr Leu Cys Glu Leu Asn Val Met Glu Gln Val Tyr Asn Leu Gly His
   145                 150                 155                 160
   Ser Thr Ile Met Gln Ser Ala Trp Lys Arg Gly Gln Lys Val Thr Ile
                   165                 170                 175
   His Gly Trp Ala Tyr Gly Ile His Asp Gly Leu Leu Arg Asp Leu Asp
               180                 185                 190
   Val Thr Ala Thr Asn Arg Glu Thr Leu Glu Gln Arg Tyr Arg His Gly
           195                 200                 205
   Ile Ser Asn Leu Lys Leu Lys His Ala Asn His Lys
       210                 215                 220
<210> 3
   <211> 663
   <212> DNA
   <213> Salmonella enterica
<220>
   <221> CDS
   <222> (1)..(663)
   <223> can Kodierregion
<400> 3
   atg aaa gac ata gat aca ctc atc agc aac aat gca cta tgg tca aaa      48
   Met Lys Asp Ile Asp Thr Leu Ile Ser Asn Asn Ala Leu Trp Ser Lys
   1               5                   10                  15
   atg ctg gtg gaa gag gac ccc gga ttt ttt gag aaa ctg gcg caa gcg      96
   Met Leu Val Glu Glu Asp Pro Gly Phe Phe Glu Lys Leu Ala Gln Ala
               20                  25                  30
   caa aaa ccg cgc ttt cta tgg att gga tgt tcc gac agc cgc gtt ccc      144
   Gln Lys Pro Arg Phe Leu Trp Ile Gly Cys Ser Asp Ser Arg Val Pro
           35                  40                  45
   gca gag cgt tta acc ggg ctt gaa ccg ggc gaa ttg ttt gtt cac cgt      192
   Ala Glu Arg Leu Thr Gly Leu Glu Pro Gly Glu Leu Phe Val His Arg
       50                  55                  60
   aat gtg gct aac ctg gtt att cac acc gat ctg aac tgt ctc tcc gtg      240
   Asn Val Ala Asn Leu Val Ile His Thr Asp Leu Asn Cys Leu Ser Val
   65                  70                  75                  80
   gtt cag tat gcg gtt gat gtt ctg gaa gtt gag cat att atc att tgc      288
   Val Gln Tyr Ala Val Asp Val Leu Glu Val Glu His Ile Ile Ile Cys
                   85                  90                  95
   ggc cac tcc ggt tgc ggc ggt atc aag gct gcg gta gaa aac cct gag      336
   Gly His Ser Gly Cys Gly Gly Ile Lys Ala Ala Val Glu Asn Pro Glu
               100                 105                 110
   ctg ggc ttg att aat aac tgg ctg ctg cat atc cgc gac atc tgg ctt      384
   Leu Gly Leu Ile Asn Asn Trp Leu Leu His Ile Arg Asp Ile Trp Leu
           115                 120                 125
   aaa cat agc tcg ctg ttg gga aaa atg ccc gaa gag caa cgt ctg gac      432
   Lys His Ser Ser Leu Leu Gly Lys Met Pro Glu Glu Gln Arg Leu Asp
       130                 135                 140
   gcg ctc tat gaa ttg aac gtc atg gag cag gtc tat aac ctg ggg cat      480
   Ala Leu Tyr Glu Leu Asn Val Met Glu Gln Val Tyr Asn Leu Gly His
   145                 150                 155                 160
   tcc acc att atg cag tca gcg tgg aaa cgc ggg cag aat gtg acc att      528
   Ser Thr Ile Met Gln Ser Ala Trp Lys Arg Gly Gln Asn Val Thr Ile
                   165                 170                 175
   cac ggc tgg gcg tac agt atc aat gat ggc ctg ctg cgc gat ctt gac      576
   His Gly Trp Ala Tyr Ser Ile Asn Asp Gly Leu Leu Arg Asp Leu Asp
               180                 185                 190
   gtc acc gcc acc aac aaa aaa acg ctg gag aac ggc tat cat aag ggt      624
   Val Thr Ala Thr Asn Lys Lys Thr Leu Glu Asn Gly Tyr His Lys Gly
           195                 200                 205
   atc tcc gcc cta agt ctg aaa tac att ccc cac caa taa                  663
   Ile Ser Ala Leu Ser Leu Lys Tyr Ile Pro His Gln
       210                 215                 220
<210> 4
   <211> 220
   <212> PRT
   <213> Salmonella enterica
<400> 4
   Met Lys Asp Ile Asp Thr Leu Ile Ser Asn Asn Ala Leu Trp Ser Lys
   1               5                   10                  15
   Met Leu Val Glu Glu Asp Pro Gly Phe Phe Glu Lys Leu Ala Gln Ala
               20                  25                  30
   Gln Lys Pro Arg Phe Leu Trp Ile Gly Cys Ser Asp Ser Arg Val Pro
           35                  40                  45
   Ala Glu Arg Leu Thr Gly Leu Glu Pro Gly Glu Leu Phe Val His Arg
       50                  55                  60
   Asn Val Ala Asn Leu Val Ile His Thr Asp Leu Asn Cys Leu Ser Val
   65                  70                  75                  80
   Val Gln Tyr Ala Val Asp Val Leu Glu Val Glu His Ile Ile Ile Cys
                   85                  90                  95
   Gly His Ser Gly Cys Gly Gly Ile Lys Ala Ala Val Glu Asn Pro Glu
               100                 105                 110
   Leu Gly Leu Ile Asn Asn Trp Leu Leu His Ile Arg Asp Ile Trp Leu
           115                 120                 125
   Lys His Ser Ser Leu Leu Gly Lys Met Pro Glu Glu Gln Arg Leu Asp
       130                 135                 140
   Ala Leu Tyr Glu Leu Asn Val Met Glu Gln Val Tyr Asn Leu Gly His
   145                 150                 155                 160
   Ser Thr Ile Met Gln Ser Ala Trp Lys Arg Gly Gln Asn Val Thr Ile
                   165                 170                 175
   His Gly Trp Ala Tyr Ser Ile Asn Asp Gly Leu Leu Arg Asp Leu Asp
               180                 185                 190
   Val Thr Ala Thr Asn Lys Lys Thr Leu Glu Asn Gly Tyr His Lys Gly
           195                 200                 205
   Ile Ser Ala Leu Ser Leu Lys Tyr Ile Pro His Gln
       210                 215                 220
<210> 5
   <211> 654
   <212> DNA
   <213> Photorhabdus luminescens
<220>
   <221> CDS
   <222> (1)..(654)
   <223> can Kodierregion
<400> 5
   atg atg aaa aaa ata gaa gag ctg ctt gcc aat aac aag cag tgg tca      48
   Met Met Lys Lys Ile Glu Glu Leu Leu Ala Asn Asn Lys Gln Trp Ser
   1               5                   10                  15
   gaa tcg gtt aac gaa gaa aat cca att ttt ttc aaa gaa tta gca aaa      96
   Glu Ser Val Asn Glu Glu Asn Pro Ile Phe Phe Lys Glu Leu Ala Lys
               20                  25                  30
   tca caa aaa cct cat ttc tta tgg att ggt tgt tca gac agc cgg gta     144
   Ser Gln Lys Pro His Phe Leu Trp Ile Gly Cys Ser Asp Ser Arg Val
           35                  40                  45
   cca gca gaa aaa cta att gat gcc gcc ccc ggt gac tta ttt gtt cac     192
   Pro Ala Glu Lys Leu Ile Asp Ala Ala Pro Gly Asp Leu Phe Val His
       50                  55                  60
   aga aac gtt gca aac ctg gtt att cat act gac tta aat tgt ctt tca     240
   Arg Asn Val Ala Asn Leu Val Ile His Thr Asp Leu Asn Cys Leu Ser
   65                  70                  75                  80
   gtt att caa tat gca gta gat gta ctg gaa gtt gaa cat atc att atc     288
   Val Ile Gln Tyr Ala Val Asp Val Leu Glu Val Glu His Ile Ile Ile
                   85                  90                  95
   tgc gga cac tac ggt tgt ggt ggt atc gaa gct gca att gat ggc acc     336
   Cys Gly His Tyr Gly Cys Gly Gly Ile Glu Ala Ala Ile Asp Gly Thr
               100                 105                 110
   gaa ctg gga ttg att aac aac tgg ctg cta cat att cgt gat ctt tgg     384
   Glu Leu Gly Leu Ile Asn Asn Trp Leu Leu His Ile Arg Asp Leu Trp
           115                 120                 125
   tat aaa cac agt tct atg cta ggt gaa tta ccc cct aat gag cgt ctg     432
   Tyr Lys His Ser Ser Met Leu Gly Glu Leu Pro Pro Asn Glu Arg Leu
       130                 135                 140
   aac cgt ctg tgc gaa att aac gtt gtt gag caa gta tat aac ctt ggt     480
   Asn Arg Leu Cys Glu Ile Asn Val Val Glu Gln Val Tyr Asn Leu Gly
   145                 150                 155                 160
   cac tca acc att atg caa gca gca tgg aaa cga ggc caa aaa gtg atg     528
   His Ser Thr Ile Met Gln Ala Ala Trp Lys Arg Gly Gln Lys Val Met
                   165                 170                 175
   atc cac ggt tgg gtt tac ggt atc cat aat ggt caa ttg caa gat ctg     576
   Ile His Gly Trp Val Tyr Gly Ile His Asn Gly Gln Leu Gln Asp Leu
               180                 185                 190
   gaa gtc acg gct gac agc cgt gaa aaa ctg gag ctt gcc tat cgc cag     624
   Glu Val Thr Ala Asp Ser Arg Glu Lys Leu Glu Leu Ala Tyr Arg Gln
           195                 200                 205
   aca ata tcc aaa ctg acg cat aag aag tga                             654
   Thr Ile Ser Lys Leu Thr His Lys Lys
       210                 215
<210> 6
   <211> 217
   <212> PRT
   <213> Photorhabdus luminescens
<400> 6
   Met Met Lys Lys Ile Glu Glu Leu Leu Ala Asn Asn Lys Gln Trp Ser
   1               5                   10                  15
   Glu Ser Val Asn Glu Glu Asn Pro Ile Phe Phe Lys Glu Leu Ala Lys
               20                  25                  30
   Ser Gln Lys Pro His Phe Leu Trp Ile Gly Cys Ser Asp Ser Arg Val
           35                  40                  45
   Pro Ala Glu Lys Leu Ile Asp Ala Ala Pro Gly Asp Leu Phe Val His
       50                  55                  60
   Arg Asn Val Ala Asn Leu Val Ile His Thr Asp Leu Asn Cys Leu Ser
   65                  70                  75                  80
   Val Ile Gln Tyr Ala Val Asp Val Leu Glu Val Glu His Ile Ile Ile
                   85                  90                  95
   Cys Gly His Tyr Gly Cys Gly Gly Ile Glu Ala Ala Ile Asp Gly Thr
               100                 105                 110
   Glu Leu Gly Leu Ile Asn Asn Trp Leu Leu His Ile Arg Asp Leu Trp
           115                 120                 125
   Tyr Lys His Ser Ser Met Leu Gly Glu Leu Pro Pro Asn Glu Arg Leu
       130                 135                 140
   Asn Arg Leu Cys Glu Ile Asn Val Val Glu Gln Val Tyr Asn Leu Gly
   145                 150                 155                 160
   His Ser Thr Ile Met Gln Ala Ala Trp Lys Arg Gly Gln Lys Val Met
                   165                 170                 175
   Ile His Gly Trp Val Tyr Gly Ile His Asn Gly Gln Leu Gln Asp Leu
               180                 185                 190
   Glu Val Thr Ala Asp Ser Arg Glu Lys Leu Glu Leu Ala Tyr Arg Gln
           195                 200                 205
   Thr Ile Ser Lys Leu Thr His Lys Lys
       210                 215
<210> 7
   <211> 660
   <212> DNA
   <213> Escherichia coli
<220>
   <221> CDS
   <222> (1)..(660)
   <223> cynT Kodierregion
<400> 7
   gtg aaa gag att att gat gga ttc ctt aaa ttc cag cgc gag gca ttt      48
   Val Lys Glu Ile Ile Asp Gly Phe Leu Lys Phe Gln Arg Glu Ala Phe
   1               5                   10                  15
   ccg aag cgg gaa gcc ttg ttt aaa cag ctg gcg aca cag caa agc ccg      96
   Pro Lys Arg Glu Ala Leu Phe Lys Gln Leu Ala Thr Gln Gln Ser Pro
               20                  25                  30
   cgc aca ctt ttt atc tcc tgc tcc gac agc cgt ctg gtc cct gag ctg     144
   Arg Thr Leu Phe Ile Ser Cys Ser Asp Ser Arg Leu Val Pro Glu Leu
           35                  40                  45
   gtg acg caa cgt gag cct ggc gat ctg ttc gtt att cgc aac gcg ggc     192
   Val Thr Gln Arg Glu Pro Gly Asp Leu Phe Val Ile Arg Asn Ala Gly
       50                  55                  60
   aat atc gtc cct tcc tac ggg ccg gaa ccc ggt ggc gtt tct gct tcg     240
   Asn Ile Val Pro Ser Tyr Gly Pro Glu Pro Gly Gly Val Ser Ala Ser
   65                  70                  75                  80
   gtg gag tat gcc gtc gct gcg ctt cgg gta tct gac att gtg att tgt     288
   Val Glu Tyr Ala Val Ala Ala Leu Arg Val Ser Asp Ile Val Ile Cys
                   85                  90                  95
   ggt cat tcc aac tgt ggc gcg atg acc gcc att gcc agc tgt cag tgc     336
   Gly His Ser Asn Cys Gly Ala Met Thr Ala Ile Ala Ser Cys Gln Cys
               100                 105                 110
   atg gac cat atg cct gcc gtc tcc cac tgg ctg cgt tat gcc gat tca     384
   Met Asp His Met Pro Ala Val Ser His Trp Leu Arg Tyr Ala Asp Ser
           115                 120                 125
   gcc cgc gtc gtt aat gag gcg cgc ccg cat tcc gat tta ccg tca aaa     432
   Ala Arg Val Val Asn Glu Ala Arg Pro His Ser Asp Leu Pro Ser Lys
       130                 135                 140
   gct gcg gcg atg gta cgt gaa aac gtc att gct cag ttg gct aat ttg     480
   Ala Ala Ala Met Val Arg Glu Asn Val Ile Ala Gln Leu Ala Asn Leu
   145                 150                 155                 160
   caa act cat cca tcg gtg cgc ctg gcg ctc gaa gag ggg cgg atc gcc     528
   Gln Thr His Pro Ser Val Arg Leu Ala Leu Glu Glu Gly Arg Ile Ala
                   165                 170                 175
   ctg cac ggc tgg gtc tac gac att gaa agc ggc agc atc gca gct ttt     576
   Leu His Gly Trp Val Tyr Asp Ile Glu Ser Gly Ser Ile Ala Ala Phe
               180                 185                 190
   gac ggc gca acc cgc cag ttt gtg cca ctg gcc gct aat cct cgc gtt     624
   Asp Gly Ala Thr Arg Gln Phe Val Pro Leu Ala Ala Asn Pro Arg Val
           195                 200                 205
   tgt gcc ata ccg cta cgc caa ccg acc gca gcg taa                     660
   Cys Ala Ile Pro Leu Arg Gln Pro Thr Ala Ala
       210                 215
<210> 8
   <211> 219
   <212> PRT
   <213> Escherichia coli
<400> 8
   Val Lys Glu Ile Ile Asp Gly Phe Leu Lys Phe Gln Arg Glu Ala Phe
   1               5                   10                  15
   Pro Lys Arg Glu Ala Leu Phe Lys Gln Leu Ala Thr Gln Gln Ser Pro
               20                  25                  30
   Arg Thr Leu Phe Ile Ser Cys Ser Asp Ser Arg Leu Val Pro Glu Leu
           35                  40                  45
   Val Thr Gln Arg Glu Pro Gly Asp Leu Phe Val Ile Arg Asn Ala Gly
       50                  55                  60
   Asn Ile Val Pro Ser Tyr Gly Pro Glu Pro Gly Gly Val Ser Ala Ser
   65                  70                  75                  80
   Val Glu Tyr Ala Val Ala Ala Leu Arg Val Ser Asp Ile Val Ile Cys
                   85                  90                  95
   Gly His Ser Asn Cys Gly Ala Met Thr Ala Ile Ala Ser Cys Gln Cys
               100                 105                 110
   Met Asp His Met Pro Ala Val Ser His Trp Leu Arg Tyr Ala Asp Ser
           115                 120                 125
   Ala Arg Val Val Asn Glu Ala Arg Pro His Ser Asp Leu Pro Ser Lys
       130                 135                 140
   Ala Ala Ala Met Val Arg Glu Asn Val Ile Ala Gln Leu Ala Asn Leu
   145                 150                 155                 160
   Gln Thr His Pro Ser Val Arg Leu Ala Leu Glu Glu Gly Arg Ile Ala
                   165                 170                 175
   Leu His Gly Trp Val Tyr Asp Ile Glu Ser Gly Ser Ile Ala Ala Phe
               180                 185                 190
   Asp Gly Ala Thr Arg Gln Phe Val Pro Leu Ala Ala Asn Pro Arg Val
           195                 200                 205
   Cys Ala Ile Pro Leu Arg Gln Pro Thr Ala Ala
       210                 215
<210> 9
   <211> 654
   <212> DNA
   <213> Serratia proteamaculans
<220>
   <221> CDS
   <222> (1)..(654)
   <223> cynT Kodierregion
<400> 9
   atg aaa gag gtt att gaa ggc ttt ttg aag ttc cag cgt gag gcg ttt      48
   Met Lys Glu Val Ile Glu Gly Phe Leu Lys Phe Gln Arg Glu Ala Phe
   1               5                   10                  15
   gtc gaa cgc acg gca ctg ttc cag cgg ctg gcg acc cag cag agc cca      96
   Val Glu Arg Thr Ala Leu Phe Gln Arg Leu Ala Thr Gln Gln Ser Pro
               20                  25                  30
   cgc acc ctg ttt atc tcc tgt tcc gac agc cgt ctg gtg ccg gag ctg     144
   Arg Thr Leu Phe Ile Ser Cys Ser Asp Ser Arg Leu Val Pro Glu Leu
           35                  40                  45
   att acc cag cgc gaa ccg ggc gac ctg ttt gtg atc cgc aat gcc ggc     192
   Ile Thr Gln Arg Glu Pro Gly Asp Leu Phe Val Ile Arg Asn Ala Gly
       50                  55                  60
   aat atc gtg cct tcg ttc ggc ccg gag ccg ggt ggc gtc tcc gca tca     240
   Asn Ile Val Pro Ser Phe Gly Pro Glu Pro Gly Gly Val Ser Ala Ser
   65                  70                  75                  80
   gta gaa tat gcc gtc tct gca ctc ggc gtt gaa gat atc gtg atc tgc     288
   Val Glu Tyr Ala Val Ser Ala Leu Gly Val Glu Asp Ile Val Ile Cys
                   85                  90                  95
   gga cat tcc gac tgt ggc gca atg act gcc atc gcc acc tgc cag tgt     336
   Gly His Ser Asp Cys Gly Ala Met Thr Ala Ile Ala Thr Cys Gln Cys
               100                 105                 110
   ctg caa cat atg ccg acc gtg gct aac tgg ctg cgc tat gcc gac tcc     384
   Leu Gln His Met Pro Thr Val Ala Asn Trp Leu Arg Tyr Ala Asp Ser
           115                 120                 125
   gcc aag gtg gtc aat cag gct tat cag cat gca tcc gaa aac gag aaa     432
   Ala Lys Val Val Asn Gln Ala Tyr Gln His Ala Ser Glu Asn Glu Lys
       130                 135                 140
   gtc agt tcg atg gtg cgt gag aac gtc atc gca cag ctg aac aat atc     480
   Val Ser Ser Met Val Arg Glu Asn Val Ile Ala Gln Leu Asn Asn Ile
   145                 150                 155                 160
   aag acc cac ccg tcg gtg gca ctg gcg ctt gaa caa ggc cgg ttg aag     528
   Lys Thr His Pro Ser Val Ala Leu Ala Leu Glu Gln Gly Arg Leu Lys
                   165                 170                 175
   ctg cac ggt tgg gtg tat gac atc gcc agc ggc ggt att gaa gcg ctg     576
   Leu His Gly Trp Val Tyr Asp Ile Ala Ser Gly Gly Ile Glu Ala Leu
               180                 185                 190
   gat ggc gaa acc cgc cgt ttt att ccg ctg gca acc aat cct gag gtc     624
   Asp Gly Glu Thr Arg Arg Phe Ile Pro Leu Ala Thr Asn Pro Glu Val
           195                 200                 205
   acg gca acc cct gcc gta tct cgc ttt taa                             654
   Thr Ala Thr Pro Ala Val Ser Arg Phe
       210                 215
<210> 10
   <211> 217
   <212> PRT
   <213> Serratia proteamaculans
<400> 10
   Met Lys Glu Val Ile Glu Gly Phe Leu Lys Phe Gln Arg Glu Ala Phe
   1               5                   10                  15
   Val Glu Arg Thr Ala Leu Phe Gln Arg Leu Ala Thr Gln Gln Ser Pro
               20                  25                  30
   Arg Thr Leu Phe Ile Ser Cys Ser Asp Ser Arg Leu Val Pro Glu Leu
           35                  40                  45
   Ile Thr Gln Arg Glu Pro Gly Asp Leu Phe Val Ile Arg Asn Ala Gly
       50                  55                  60
   Asn Ile Val Pro Ser Phe Gly Pro Glu Pro Gly Gly Val Ser Ala Ser
   65                  70                  75                  80
   Val Glu Tyr Ala Val Ser Ala Leu Gly Val Glu Asp Ile Val Ile Cys
                   85                  90                  95
   Gly His Ser Asp Cys Gly Ala Met Thr Ala Ile Ala Thr Cys Gln Cys
               100                 105                 110
   Leu Gln His Met Pro Thr Val Ala Asn Trp Leu Arg Tyr Ala Asp Ser
           115                 120                 125
   Ala Lys Val Val Asn Gln Ala Tyr Gln His Ala Ser Glu Asn Glu Lys
       130                 135                 140
   Val Ser Ser Met Val Arg Glu Asn Val Ile Ala Gln Leu Asn Asn Ile
   145                 150                 155                 160
   Lys Thr His Pro Ser Val Ala Leu Ala Leu Glu Gln Gly Arg Leu Lys
                   165                 170                 175
   Leu His Gly Trp Val Tyr Asp Ile Ala Ser Gly Gly Ile Glu Ala Leu
               180                 185                 190
   Asp Gly Glu Thr Arg Arg Phe Ile Pro Leu Ala Thr Asn Pro Glu Val
           195                 200                 205
   Thr Ala Thr Pro Ala Val Ser Arg Phe
       210                 215
<210> 11
   <211> 660
   <212> DNA
   <213> Photorhabdus luminescens
<220>
   <221> CDS
   <222> (1)..(660)
   <223> cynT Kodierregion
<400> 11
   atg cag gat att att gaa ggc ttt tta caa ttc cag cgc gag gta ttt        48
   Met Gln Asp Ile Ile Glu Gly Phe Leu Gln Phe Gln Arg Glu Val Phe
   1               5                   10                  15
   cca aag cgt tgc gag ctt ttc aag cgg ctt gcc acc agc caa aat ccg        96
   Pro Lys Arg Cys Glu Leu Phe Lys Arg Leu Ala Thr Ser Gln Asn Pro
               20                  25                  30
   cgt act ttg ttt atc acg tgc tcc gac agc cgt gtg gtt cca gag ctt       144
   Arg Thr Leu Phe Ile Thr Cys Ser Asp Ser Arg Val Val Pro Glu Leu
           35                  40                  45
   ctg act cag agt gag cca ggt gat ctg ttc gtg ata cgc aat gcg ggt       192
   Leu Thr Gln Ser Glu Pro Gly Asp Leu Phe Val Ile Arg Asn Ala Gly
       50                  55                  60
   aac atc gtt ccg tcg tac ggt gca gaa tct ggc ggt gtt tcc gct acc       240
   Asn Ile Val Pro Ser Tyr Gly Ala Glu Ser Gly Gly Val Ser Ala Thr
   65                  70                  75                  80
   gtt gag tat gcg gtc gct gtg ctt gga gtc acc gac att gtg atc tgc       288
   Val Glu Tyr Ala Val Ala Val Leu Gly Val Thr Asp Ile Val Ile Cys
                   85                  90                  95
   gga cat tca gat tgt ggt gcc atg acc aca att gca agc tgt aaa tgt       336
   Gly His Ser Asp Cys Gly Ala Met Thr Thr Ile Ala Ser Cys Lys Cys
               100                 105                 110
   atg aat cac ctg ccc gcg att aca agc tgg tta cgc tat gcg gat tgt       384
   Met Asn His Leu Pro Ala Ile Thr Ser Trp Leu Arg Tyr Ala Asp Cys
           115                 120                 125
   gcg aaa gcg att agt cag gca cgt gtg cac gtt aac gag act gct caa       432
   Ala Lys Ala Ile Ser Gln Ala Arg Val His Val Asn Glu Thr Ala Gln
       130                 135                 140
   att gat tcg atg gtg cgt gaa aac gta atc gct caa att gct aat atc       480
   Ile Asp Ser Met Val Arg Glu Asn Val Ile Ala Gln Ile Ala Asn Ile
   145                 150                 155                 160
   aaa acc cac ccg tcc gtg gca ctc ggg ctg gaa cag agg cac ttg aat       528
   Lys Thr His Pro Ser Val Ala Leu Gly Leu Glu Gln Arg His Leu Asn
                   165                 170                 175
   cta cat ggc tgg att tac gac ata gag gct ggt tcg atc gat gct tta       576
   Leu His Gly Trp Ile Tyr Asp Ile Glu Ala Gly Ser Ile Asp Ala Leu
               180                 185                 190
   gac ggc ttg gcc ggt caa ttt gtt tcc ctt gcg gac tat ccg cat gtt       624
   Asp Gly Leu Ala Gly Gln Phe Val Ser Leu Ala Asp Tyr Pro His Val
           195                 200                 205
   tct gca acg caa tcc gtg ttt cac cac ggc att taa                       660
   Ser Ala Thr Gln Ser Val Phe His His Gly Ile
       210                 215
<210> 12
   <211> 219
   <212> PRT
   <213> Photorhabdus luminescens
<400> 12
   Met Gln Asp Ile Ile Glu Gly Phe Leu Gln Phe Gln Arg Glu Val Phe
   1               5                   10                  15
   Pro Lys Arg Cys Glu Leu Phe Lys Arg Leu Ala Thr Ser Gln Asn Pro
               20                  25                  30
   Arg Thr Leu Phe Ile Thr Cys Ser Asp Ser Arg Val Val Pro Glu Leu
           35                  40                  45
   Leu Thr Gln Ser Glu Pro Gly Asp Leu Phe Val Ile Arg Asn Ala Gly
       50                  55                  60
   Asn Ile Val Pro Ser Tyr Gly Ala Glu Ser Gly Gly Val Ser Ala Thr
   65                  70                  75                  80
   Val Glu Tyr Ala Val Ala Val Leu Gly Val Thr Asp Ile Val Ile Cys
                   85                  90                  95
   Gly His Ser Asp Cys Gly Ala Met Thr Thr Ile Ala Ser Cys Lys Cys
               100                 105                 110
   Met Asn His Leu Pro Ala Ile Thr Ser Trp Leu Arg Tyr Ala Asp Cys
           115                 120                 125
   Ala Lys Ala Ile Ser Gln Ala Arg Val His Val Asn Glu Thr Ala Gln
       130                 135                 140
   Ile Asp Ser Met Val Arg Glu Asn Val Ile Ala Gln Ile Ala Asn Ile
   145                 150                 155                 160
   Lys Thr His Pro Ser Val Ala Leu Gly Leu Glu Gln Arg His Leu Asn
                   165                 170                 175
   Leu His Gly Trp Ile Tyr Asp Ile Glu Ala Gly Ser Ile Asp Ala Leu
               180                 185                 190
   Asp Gly Leu Ala Gly Gln Phe Val Ser Leu Ala Asp Tyr Pro His Val
           195                 200                 205
   Ser Ala Thr Gln Ser Val Phe His His Gly Ile
       210                 215
<210> 13
   <211> 2463
   <212> DNA
   <213> Escherichia coli
<220>
   <221> CDS
   <222> (1)..(2963)
   <223> thrA-Kodierregion
<400> 13
   atg cga gtg ttg aag ttc ggc ggt aca tca gtg gca aat gca gaa cgt        48
   Met Arg Val Leu Lys Phe Gly Gly Thr Ser Val Ala Asn Ala Glu Arg
   1               5                   10                  15
   ttt ctg cgt gtt gcc gat att ctg gaa agc aat gcc agg cag ggg cag        96
   Phe Leu Arg Val Ala Asp Ile Leu Glu Ser Asn Ala Arg Gln Gly Gln
               20                  25                  30
   gtg gcc acc gtc ctc tct gcc ccc gcc aaa atc acc aac cac ctg gtg       144
   Val Ala Thr Val Leu Ser Ala Pro Ala Lys Ile Thr Asn His Leu Val
           35                  40                  45
   gcg atg att gaa aaa acc att agc ggc cag gat gct tta ccc aat atc       192
   Ala Met Ile Glu Lys Thr Ile Ser Gly Gln Asp Ala Leu Pro Asn Ile
       50                  55                  60
   agc gat gcc gaa cgt att ttt gcc gaa ctt ttg acg gga ctc gcc gcc       240
   Ser Asp Ala Glu Arg Ile Phe Ala Glu Leu Leu Thr Gly Leu Ala Ala
   65                  70                  75                  80
   gcc cag ccg ggg ttc ccg ctg gcg caa ttg aaa act ttc gtc gat cag       288
   Ala Gln Pro Gly Phe Pro Leu Ala Gln Leu Lys Thr Phe Val Asp Gln
                   85                  90                  95
   gaa ttt gcc caa ata aaa cat gtc ctg cat ggc att agt ttg ttg ggg       336
   Glu Phe Ala Gln Ile Lys His Val Leu His Gly Ile Ser Leu Leu Gly
               100                 105                 110
   cag tgc ccg gat agc atc aac gct gcg ctg att tgc cgt ggc gag aaa       384
   Gln Cys Pro Asp Ser Ile Asn Ala Ala Leu Ile Cys Arg Gly Glu Lys
           115                 120                 125
   atg tcg atc gcc att atg gcc ggc gta tta gaa gcg cgc ggt cac aac       432
   Met Ser Ile Ala Ile Met Ala Gly Val Leu Glu Ala Arg Gly His Asn
       130                 135                 140
   gtt act gtt atc gat ccg gtc gaa aaa ctg ctg gca gtg ggg cat tac       480
   Val Thr Val Ile Asp Pro Val Glu Lys Leu Leu Ala Val Gly His Tyr
   145                 150                 155                 160
   ctc gaa tct acc gtc gat att gct gag tcc acc cgc cgt att gcg gca       528
   Leu Glu Ser Thr Val Asp Ile Ala Glu Ser Thr Arg Arg Ile Ala Ala
                   165                 170                 175
   agc cgc att ccg gct gat cac atg gtg ctg atg gca ggt ttc acc gcc       576
   Ser Arg Ile Pro Ala Asp His Met Val Leu Met Ala Gly Phe Thr Ala
               180                 185                 190
   ggt aat gaa aaa ggc gaa ctg gtg gtg ctt gga cgc aac ggt tcc gac       624
   Gly Asn Glu Lys Gly Glu Leu Val Val Leu Gly Arg Asn Gly Ser Asp
           195                 200                 205
   tac tct gct gcg gtg ctg gct gcc tgt tta cgc gcc gat tgt tgc gag      672
   Tyr Ser Ala Ala Val Leu Ala Ala Cys Leu Arg Ala Asp Cys Cys Glu
       210                 215                 220
   att tgg acg gac gtt gac ggg gtc tat acc tgc gac ccg cgt cag gtg      720
   Ile Trp Thr Asp Val Asp Gly Val Tyr Thr Cys Asp Pro Arg Gln Val
   225                 230                 235                 240
   ccc gat gcg agg ttg ttg aag tcg atg tcc tac cag gaa gcg atg gag      768
   Pro Asp Ala Arg Leu Leu Lys Ser Met Ser Tyr Gln Glu Ala Met Glu
                   245                 250                 255
   ctt tcc tac ttc ggc gct aaa gtt ctt cac ccc cgc acc att acc ccc      816
   Leu Ser Tyr Phe Gly Ala Lys Val Leu His Pro Arg Thr Ile Thr Pro
               260                 265                 270
   atc gcc cag ttc cag atc cct tgc ctg att aaa aat acc gga aat cct      864
   Ile Ala Gln Phe Gln Ile Pro Cys Leu Ile Lys Asn Thr Gly Asn Pro
           275                 280                 285
   caa gca cca ggt acg ctc att ggt gcc agc cgt gat gaa gac gaa tta      912
   Gln Ala Pro Gly Thr Leu Ile Gly Ala Ser Arg Asp Glu Asp Glu Leu
       290                 295                 300
   ccg gtc aag ggc att tcc aat ctg aat aac atg gca atg ttc agc gtt      960
   Pro Val Lys Gly Ile Ser Asn Leu Asn Asn Met Ala Met Phe Ser Val
   305                 310                 315                 320
   tct ggt ccg ggg atg aaa ggg atg gtc ggc atg gcg gcg cgc gtc ttt     1008
   Ser Gly Pro Gly Met Lys Gly Met Val Gly Met Ala Ala Arg Val Phe
                   325                 330                 335
   gca gcg atg tca cgc gcc cgt att tcc gtg gtg ctg att acg caa tca     1056
   Ala Ala Met Ser Arg Ala Arg Ile Ser Val Val Leu Ile Thr Gln Ser
               340                 345                 350
   tct tcc gaa tac agc atc agt ttc tgc gtt cca caa agc gac tgt gtg     1104
   Ser Ser Glu Tyr Ser Ile Ser Phe Cys Val Pro Gln Ser Asp Cys Val
           355                 360                 365
   cga gct gaa cgg gca atg cag gaa gag ttc tac ctg gaa ctg aaa gaa     1152
   Arg Ala Glu Arg Ala Met Gln Glu Glu Phe Tyr Leu Glu Leu Lys Glu
       370                 375                 380
   ggc tta ctg gag ccg ctg gca gtg acg gaa cgg ctg gcc att atc tcg     1200
   Gly Leu Leu Glu Pro Leu Ala Val Thr Glu Arg Leu Ala Ile Ile Ser
   385                 390                 395                 400
   gtg gta ggt gat ggt atg cgc acc ttg cgt ggg atc tcg gcg aaa ttc     1248
   Val Val Gly Asp Gly Met Arg Thr Leu Arg Gly Ile Ser Ala Lys Phe
                   405                 410                 415
   ttt gcc gca ctg gcc cgc gcc aat atc aac att gtc gcc att gct cag     1296
   Phe Ala Ala Leu Ala Arg Ala Asn Ile Asn Ile Val Ala Ile Ala Gln
               420                 425                 430
   gga tct tct gaa cgc tca atc tct gtc gtg gta aat aac gat gat gcg     1344
   Gly Ser Ser Glu Arg Ser Ile Ser Val Val Val Asn Asn Asp Asp Ala
           435                 440                 445
   acc act ggc gtg cgc gtt act cat cag atg ctg ttc aat acc gat cag     1392
   Thr Thr Gly Val Arg Val Thr His Gln Met Leu Phe Asn Thr Asp Gln
   450 455 460
   gtt atc gaa gtg ttt gtg att ggc gtc ggt ggc gtt ggc ggt gcg ctg 1440
   Val Ile Glu Val Phe Val Ile Gly Val Gly Gly Val Gly Gly Ala Leu
   465 470 475 480
   ctg gag caa ctg aag cgt cag caa agc tgg ctg aag aat aaa cat atc 1488
   Leu Glu Gln Leu Lys Arg Gln Gln Ser Trp Leu Lys Asn Lys His Ile
   485 490 495
   gac tta cgt gtc tgc ggt gtt gcc aac tcg aag gct ctg ctc acc aat 1536
   Asp Leu Arg Val Cys Gly Val Ala Asn Ser Lys Ala Leu Leu Thr Asn
   500 505 510
   gta cat ggc ctt aat ctg gaa aac tgg cag gaa gaa ctg gcg caa gcc 1584
   Val His Gly Leu Asn Leu Glu Asn Trp Gln Glu Glu Leu Ala Gln Ala
   515 520 525
   aaa gag ccg ttt aat ctc ggg cgc tta att cgc ctc gtg aaa gaa tat 1632
   Lys Glu Pro Phe Asn Leu Gly Arg Leu Ile Arg Leu Val Lys Glu Tyr
   530 535 540
   cat ctg ctg aac ccg gtc att gtt gac tgc act tcc agc cag gca gtg 1680
   His Leu Leu Asn Pro Val Ile Val Asp Cys Thr Ser Ser Gln Ala Val
   545 550 555 560
   gcg gat caa tat gcc gac ttc ctg cgc gaa ggt ttc cac gtt gtc acg 1728
   Ala Asp Gln Tyr Ala Asp Phe Leu Arg Glu Gly Phe His Val Val Thr
   565 570 575
   ccg aac aaa aag gcc aac acc tcg tcg atg gat tac tac cat cag ttg 1776
   Pro Asn Lys Lys Ala Asn Thr Ser Ser Met Asp Tyr Tyr His Gln Leu
   580 585 590
   cgt tat gcg gcg gaa aaa tcg cgg cgt aaa ttc ctc tat gac acc aac 1824
   Arg Tyr Ala Ala Glu Lys Ser Arg Arg Lys Phe Leu Tyr Asp Thr Asn
   595 600 605
   gtt ggg gct gga tta ccg gtt att gag aac ctg caa aat ctg ctc aat 1872
   Val Gly Ala Gly Leu Pro Val Ile Glu Asn Leu Gln Asn Leu Leu Asn
   610 615 620
   gca ggt gat gaa ttg atg aag ttc tcc ggc att ctt tct ggt tcg ctt 1920
   Ala Gly Asp Glu Leu Met Lys Phe Ser Gly Ile Leu Ser Gly Ser Leu
   625 630 635 640
   tct tat atc ttc ggc aag tta gac gaa ggc atg agt ttc tcc gag gcg 1968
   Ser Tyr Ile Phe Gly Lys Leu Asp Glu Gly Met Ser Phe Ser Glu Ala
   645 650 655
   acc acg ctg gcg cgg gaa atg ggt tat acc gaa ccg gac ccg cga gat 2016
   Thr Thr Leu Ala Arg Glu Met Gly Tyr Thr Glu Pro Asp Pro Arg Asp
   660 665 670
   gat ctt tct ggt atg gat gtg gcg cgt aaa cta ttg att ctc gct cgt 2064
   Asp Leu Ser Gly Met Asp Val Ala Arg Lys Leu Leu Ile Leu Ala Arg
   675 680 685
   gaa acg gga cgt gaa ctg gag ctg gcg gat att gaa att gaa cct gtg 2112
   Glu Thr Gly Arg Glu Leu Glu Leu Ala Asp Ile Glu Ile Glu Pro Val
   690 695 700
   ctg ccc gca gag ttt aac gcc gag ggt gat gtt gcc gct ttt atg gcg      2160
   Leu Pro Ala Glu Phe Asn Ala Glu Gly Asp Val Ala Ala Phe Met Ala
   705                 710                 715                 720
   aat ctg tca caa ctc gac gat ctc ttt gcc gcg cgc gtg gcg aag gcc      2208
   Asn Leu Ser Gln Leu Asp Asp Leu Phe Ala Ala Arg Val Ala Lys Ala
                   725                 730                 735
   cgt gat gaa gga aaa gtt ttg cgc tat gtt ggc aat att gat gaa gat      2256
   Arg Asp Glu Gly Lys Val Leu Arg Tyr Val Gly Asn Ile Asp Glu Asp
               740                 745                 750
   ggc gtc tgc cgc gtg aag att gcc gaa gtg gat ggt aat gat ccg ctg      2304
   Gly Val Cys Arg Val Lys Ile Ala Glu Val Asp Gly Asn Asp Pro Leu
           755                 760                 765
   ttc aaa gtg aaa aat ggc gaa aac gcc ctg gcc ttc tat agc cac tat      2352
   Phe Lys Val Lys Asn Gly Glu Asn Ala Leu Ala Phe Tyr Ser His Tyr
       770                 775                 780
   tat cag ccg ctg ccg ttg gta ctg cgc gga tat ggt gcg ggc aat gac      2400
   Tyr Gln Pro Leu Pro Leu Val Leu Arg Gly Tyr Gly Ala Gly Asn Asp
   785                 790                 795                 800
   gtt aca gct gcc ggt gtc ttt gct gat ctg cta cgt acc ctc tca tgg      2448
   Val Thr Ala Ala Gly Val Phe Ala Asp Leu Leu Arg Thr Leu Ser Trp
                   805                 810                 815
   aag tta gga gtc tga                                                  2463
   Lys Leu Gly Val
               820
<210> 14
   <211> 820
   <212> PRT
   <213> Escherichia coli
<400> 14
   Met Arg Val Leu Lys Phe Gly Gly Thr Ser Val Ala Asn Ala Glu Arg
   1               5                   10                  15
   Phe Leu Arg Val Ala Asp Ile Leu Glu Ser Asn Ala Arg Gln Gly Gln
               20                  25                  30
   Val Ala Thr Val Leu Ser Ala Pro Ala Lys Ile Thr Asn His Leu Val
           35                  40                  45
   Ala Met Ile Glu Lys Thr Ile Ser Gly Gln Asp Ala Leu Pro Asn Ile
       50                  55                  60
   Ser Asp Ala Glu Arg Ile Phe Ala Glu Leu Leu Thr Gly Leu Ala Ala
   65                  70                  75                  80
   Ala Gln Pro Gly Phe Pro Leu Ala Gln Leu Lys Thr Phe Val Asp Gln
   85 90 95
   Glu Phe Ala Gln Ile Lys His Val Leu His Gly Ile Ser Leu Leu Gly
   100 105 110
   Gln Cys Pro Asp Ser Ile Asn Ala Ala Leu Ile Cys Arg Gly Glu Lys
   115 120 125
   Met Ser Ile Ala Ile Met Ala Gly Val Leu Glu Ala Arg Gly His Asn
   130 135 140
   Val Thr Val Ile Asp Pro Val Glu Lys Leu Leu Ala Val Gly His Tyr
   145 150 155 160
   Leu Glu Ser Thr Val Asp Ile Ala Glu Ser Thr Arg Arg Ile Ala Ala
   165 170 175
   Ser Arg Ile Pro Ala Asp His Met Val Leu Met Ala Gly Phe Thr Ala
   180 185 190
   Gly Asn Glu Lys Gly Glu Leu Val Val Leu Gly Arg Asn Gly Ser Asp
   195 200 205
   Tyr Ser Ala Ala Val Leu Ala Ala Cys Leu Arg Ala Asp Cys Cys Glu
   210 215 220
   Ile Trp Thr Asp Val Asp Gly Val Tyr Thr Cys Asp Pro Arg Gln Val
   225 230 235 240
   Pro Asp Ala Arg Leu Leu Lys Ser Met Ser Tyr Gln Glu Ala Met Glu
   245 250 255
   Leu Ser Tyr Phe Gly Ala Lys Val Leu His Pro Arg Thr Ile Thr Pro
   260 265 270
   Ile Ala Gln Phe Gln Ile Pro Cys Leu Ile Lys Asn Thr Gly Asn Pro
   275 280 285
   Gln Ala Pro Gly Thr Leu Ile Gly Ala Ser Arg Asp Glu Asp Glu Leu
   290 295 300
   Pro Val Lys Gly Ile Ser Asn Leu Asn Asn Met Ala Met Phe Ser Val
   305 310 315 320
   Ser Gly Pro Gly Met Lys Gly Met Val Gly Met Ala Ala Arg Val Phe
   325 330 335
   Ala Ala Met Ser Arg Ala Arg Ile Ser Val Val Leu Ile Thr Gln Ser
   340 345 350
   Ser Ser Glu Tyr Ser Ile Ser Phe Cys Val Pro Gln Ser Asp Cys Val
   355 360 365
   Arg Ala Glu Arg Ala Met Gln Glu Glu Phe Tyr Leu Glu Leu Lys Glu
   370 375 380
   Gly Leu Leu Glu Pro Leu Ala Val Thr Glu Arg Leu Ala Ile Ile Ser
   385 390 395 400
   Val Val Gly Asp Gly Met Arg Thr Leu Arg Gly Ile Ser Ala Lys Phe
   405 410 415
   Phe Ala Ala Leu Ala Arg Ala Asn Ile Asn Ile Val Ala Ile Ala Gln
   420 425 430
   Gly Ser Ser Glu Arg Ser Ile Ser Val Val Val Asn Asn Asp Asp Ala
   435 440 445
   Thr Thr Gly Val Arg Val Thr His Gln Met Leu Phe Asn Thr Asp Gln
   450 455 460
   Val Ile Glu Val Phe Val Ile Gly Val Gly Gly Val Gly Gly Ala Leu
   465 470 475 480
   Leu Glu Gln Leu Lys Arg Gln Gln Ser Trp Leu Lys Asn Lys His Ile
   485 490 495
   Asp Leu Arg Val Cys Gly Val Ala Asn Ser Lys Ala Leu Leu Thr Asn
   500 505 510
   Val His Gly Leu Asn Leu Glu Asn Trp Gln Glu Glu Leu Ala Gln Ala
   515 520 525
   Lys Glu Pro Phe Asn Leu Gly Arg Leu Ile Arg Leu Val Lys Glu Tyr
   530 535 540
   His Leu Leu Asn Pro Val Ile Val Asp Cys Thr Ser Ser Gln Ala Val
   545 550 555 560
   Ala Asp Gln Tyr Ala Asp Phe Leu Arg Glu Gly Phe His Val Val Thr
   565 570 575
   Pro Asn Lys Lys Ala Asn Thr Ser Ser Met Asp Tyr Tyr His Gln Leu
   580 585 590
   Arg Tyr Ala Ala Glu Lys Ser Arg Arg Lys Phe Leu Tyr Asp Thr Asn
   595 600 605
   Val Gly Ala Gly Leu Pro Val Ile Glu Asn Leu Gln Asn Leu Leu Asn
   610 615 620
   Ala Gly Asp Glu Leu Met Lys Phe Ser Gly Ile Leu Ser Gly Ser Leu
   625 630 635 640
   Ser Tyr Ile Phe Gly Lys Leu Asp Glu Gly Met Ser Phe Ser Glu Ala
   645 650 655
   Thr Thr Leu Ala Arg Glu Met Gly Tyr Thr Glu Pro Asp Pro Arg Asp
   660 665 670
   Asp Leu Ser Gly Met Asp Val Ala Arg Lys Leu Leu Ile Leu Ala Arg
   675 680 685
   Glu Thr Gly Arg Glu Leu Glu Leu Ala Asp Ile Glu Ile Glu Pro Val
   690 695 700
   Leu Pro Ala Glu Phe Asn Ala Glu Gly Asp Val Ala Ala Phe Met Ala
   705 710 715 720
   Asn Leu Ser Gln Leu Asp Asp Leu Phe Ala Ala Arg Val Ala Lys Ala
   725 730 735
   Arg Asp Glu Gly Lys Val Leu Arg Tyr Val Gly Asn Ile Asp Glu Asp
   740 745 750
   Gly Val Cys Arg Val Lys Ile Ala Glu Val Asp Gly Asn Asp Pro Leu
   755 760 765
   Phe Lys Val Lys Asn Gly Glu Asn Ala Leu Ala Phe Tyr Ser His Tyr
   770 775 780
   Tyr Gln Pro Leu Pro Leu Val Leu Arg Gly Tyr Gly Ala Gly Asn Asp
   785 790 795 800
   Val Thr Ala Ala Gly Val Phe Ala Asp Leu Leu Arg Thr Leu Ser Trp
   805 810 815
   Lys Leu Gly Val
   820
<210> 15
   <211> 2460
   <212> DNA
   <213> Serratia marcescens
<220>
   <221> CDS
   <222> (1)..(2460)
   <223> thrA-Kodierregion
<400> 15
   atg cga gtg ctg aaa ttt ggc gga acc tcg gta gcg aat gcg gaa cgt        48
   Met Arg Val Leu Lys Phe Gly Gly Thr Ser Val Ala Asn Ala Glu Arg
   1               5                   10                  15
   ttt ctg cgc gtc gcc gac atc atg gag agt aac gcg cgt cag gga cag        96
   Phe Leu Arg Val Ala Asp Ile Met Glu Ser Asn Ala Arg Gln Gly Gln
               20                  25                  30
   gta gcc acg gtc ttg tcc gcc ccc gca aag atc acc aac cat ctg gtg       144
   Val Ala Thr Val Leu Ser Ala Pro Ala Lys Ile Thr Asn His Leu Val
           35                  40                  45
   gcg atg atc gac aaa acg gtg gcg ggc cag gac att ctg ccg aat atg       192
   Ala Met Ile Asp Lys Thr Val Ala Gly Gln Asp Ile Leu Pro Asn Met
       50                  55                  60
   agc gac gcc gag cgg atc ttt gcc gac ctg ctg agc gga ctg gcg cag       240
   Ser Asp Ala Glu Arg Ile Phe Ala Asp Leu Leu Ser Gly Leu Ala Gln
   65                  70                  75                  80
   gcg ctg ccg ggc ttt gaa tac gat cgt ttg aaa ggc gtg gtc gat cag       288
   Ala Leu Pro Gly Phe Glu Tyr Asp Arg Leu Lys Gly Val Val Asp Gln
                   85                  90                  95
   gaa ttc gcg cag ctc aaa cag gtg ctg cat ggc gtc tcg ctg ctg ggg       336
   Glu Phe Ala Gln Leu Lys Gln Val Leu His Gly Val Ser Leu Leu Gly
               100                 105                 110
   cag tgc ccg gac agc gtg aac gcg gcg atc atc tgc cgt ggc gaa aag       384
   Gln Cys Pro Asp Ser Val Asn Ala Ala Ile Ile Cys Arg Gly Glu Lys
           115                 120                 125
   ctc tcc atc gcc atc atg gaa ggg gtg ttc cgc gcc aag ggt tat ccg       432
   Leu Ser Ile Ala Ile Met Glu Gly Val Phe Arg Ala Lys Gly Tyr Pro
       130                 135                 140
   gtc acg gtg atc aac ccg gtg gag aaa ctg ctg gcg cag ggc cac tac       480
   Val Thr Val Ile Asn Pro Val Glu Lys Leu Leu Ala Gln Gly His Tyr
   145                 150                 155                 160
   ctg gag tcc acc gtg gac atc gcc gag tct acg ctg cgc atc gcc gcc       528
   Leu Glu Ser Thr Val Asp Ile Ala Glu Ser Thr Leu Arg Ile Ala Ala
                   165                 170                 175
   gcg gcg atc ccg gcc gat cac atc gtg ctg atg gcc ggt ttc acc gcc       576
   Ala Ala Ile Pro Ala Asp His Ile Val Leu Met Ala Gly Phe Thr Ala
               180                 185                 190
   ggt aat gat aag ggc gag ctg gtg gtg ctg ggc cgc aac ggc tcc gac       624
   Gly Asn Asp Lys Gly Glu Leu Val Val Leu Gly Arg Asn Gly Ser Asp
           195                 200                 205
   tat tcc gcc gcg gtg ctg gcc gcc tgc ctg cgc gcc gac tgt tgc gag      672
   Tyr Ser Ala Ala Val Leu Ala Ala Cys Leu Arg Ala Asp Cys Cys Glu
       210                 215                 220
   atc tgg acc gac gtc gac ggc gtt tat acc tgc gat ccg cgc acc gtg      720
   Ile Trp Thr Asp Val Asp Gly Val Tyr Thr Cys Asp Pro Arg Thr Val
   225                 230                 235                 240
   ccg gac gcc agg tta ctg aaa tcg atg tcg tac cag gaa gcg atg gag      768
   Pro Asp Ala Arg Leu Leu Lys Ser Met Ser Tyr Gln Glu Ala Met Glu
                   245                 250                 255
   ctt tcc tat ttc ggc gcc aaa gtg tta cac ccc cgc acc atc act ccg      816
   Leu Ser Tyr Phe Gly Ala Lys Val Leu His Pro Arg Thr Ile Thr Pro
               260                 265                 270
   att gcc cag ttt caa atc cct tgc ctg att aaa aac acc tcc aac ccg      864
   Ile Ala Gln Phe Gln Ile Pro Cys Leu Ile Lys Asn Thr Ser Asn Pro
           275                 280                 285
   cag gcc ccc ggc acg ctg atc ggc aaa gac agc acc gat gcc gat atg      912
   Gln Ala Pro Gly Thr Leu Ile Gly Lys Asp Ser Thr Asp Ala Asp Met
       290                 295                 300
   ccg gtg aag ggc atc acc aac ctg aac aac atg gcg atg atc aac gtc      960
   Pro Val Lys Gly Ile Thr Asn Leu Asn Asn Met Ala Met Ile Asn Val
   305                 310                 315                 320
   tcc ggc ccg ggc atg aaa ggc atg gtc ggc atg gcc gca cgg gtc ttc     1008
   Ser Gly Pro Gly Met Lys Gly Met Val Gly Met Ala Ala Arg Val Phe
                   325                 330                 335
   gcc gtg atg tcg cgc gcc ggc att tcg gtg gtg ctg atc acc caa tcc     1056
   Ala Val Met Ser Arg Ala Gly Ile Ser Val Val Leu Ile Thr Gln Ser
               340                 345                 350
   tct tcc gaa tac agc atc agc ttc tgc gtg ccg cag ggt gaa ctg cag     1104
   Ser Ser Glu Tyr Ser Ile Ser Phe Cys Val Pro Gln Gly Glu Leu Gln
           355                 360                 365
   cgc gcc cgt cgc gcg ctg gaa gag gag ttc tat ctg gag ctg aaa gac     1152
   Arg Ala Arg Arg Ala Leu Glu Glu Glu Phe Tyr Leu Glu Leu Lys Asp
       370                 375                 380
   ggc gtg ctg gat ccg ctg gac gtg atg gaa cgc ctg gcg atc atc tcg     1200
   Gly Val Leu Asp Pro Leu Asp Val Met Glu Arg Leu Ala Ile Ile Ser
   385                 390                 395                 400
   gtg gtt ggc gac ggc atg cgc acc ctg cgc ggc att tcc gca cgc ttc     1248
   Val Val Gly Asp Gly Met Arg Thr Leu Arg Gly Ile Ser Ala Arg Phe
                   405                 410                 415
   ttc tcc gcg ctg gcg cgc gcc aac atc aat atc gtt gcc atc gcc caa     1296
   Phe Ser Ala Leu Ala Arg Ala Asn Ile Asn Ile Val Ala Ile Ala Gln
               420                 425                 430
   ggg tct tcc gaa cgt tcc att tcg gtg gtg gtc agc aat gat tcc gcc     1344
   Gly Ser Ser Glu Arg Ser Ile Ser Val Val Val Ser Asn Asp Ser Ala
           435                 440                 445
   acc acc ggc gtg cgc gtc agc cac cag atg ctg ttc aac acc gat cag      1392
   Thr Thr Gly Val Arg Val Ser His Gln Met Leu Phe Asn Thr Asp Gln
       450                 455                 460
   gtg atc gaa gtg ttc gtc atc ggc gtc ggc ggc gtc ggc ggg gcg ctg      1440
   Val Ile Glu Val Phe Val Ile Gly Val Gly Gly Val Gly Gly Ala Leu
   465                 470                 475                 480
   atc gag cag atc tat cgc cag cag ccg tgg ctg aag caa aaa cat atc      1488
   Ile Glu Gln Ile Tyr Arg Gln Gln Pro Trp Leu Lys Gln Lys His Ile
                   485                 490                 495
   gat ctg cgg gtg tgc ggc atc gcc aac tcg cgc gtg atg ctg acc aat      1536
   Asp Leu Arg Val Cys Gly Ile Ala Asn Ser Arg Val Met Leu Thr Asn
               500                 505                 510
   gtg cac ggc atc gcg ctg gac agc tgg cgc gac gcg ctg gcc ggc gcg      1584
   Val His Gly Ile Ala Leu Asp Ser Trp Arg Asp Ala Leu Ala Gly Ala
           515                 520                 525
   cag gag ccg ttc aat ctc ggc cgt ctg atc cgg ctg gtg aag gaa tat      1632
   Gln Glu Pro Phe Asn Leu Gly Arg Leu Ile Arg Leu Val Lys Glu Tyr
       530                 535                 540
   cac ctg ctg aac ccg gtg atc gtc gac tgt acc tcc agt cag gcg gtg      1680
   His Leu Leu Asn Pro Val Ile Val Asp Cys Thr Ser Ser Gln Ala Val
   545                 550                 555                 560
   gcc gat cag tac gtg gac ttc ctg gcg gac ggc ttc cat gtg gtg acg      1728
   Ala Asp Gln Tyr Val Asp Phe Leu Ala Asp Gly Phe His Val Val Thr
                   565                 570                 575
   ccg aac aaa aag gcc aac acc tcg tcg atg aac tat tat cag caa ctg      1776
   Pro Asn Lys Lys Ala Asn Thr Ser Ser Met Asn Tyr Tyr Gln Gln Leu
               580                 585                 590
   cgc gcg gcc gcc gcc ggt tcg cat cgc aag ttc ctg tac gac acc aac      1824
   Arg Ala Ala Ala Ala Gly Ser His Arg Lys Phe Leu Tyr Asp Thr Asn
           595                 600                 605
   gtc ggt gcc ggt ctg ccg gtg att gag aac ctg caa aac ctg ctg aac      1872
   Val Gly Ala Gly Leu Pro Val Ile Glu Asn Leu Gln Asn Leu Leu Asn
       610                 615                 620
   gcc ggt gat gaa ctg gta cgt ttc tcc ggt atc ctg tcc ggc tcg ctg      1920
   Ala Gly Asp Glu Leu Val Arg Phe Ser Gly Ile Leu Ser Gly Ser Leu
   625                 630                 635                 640
   tcc ttt atc ttc ggc aag ctg gac gaa ggg ctg tcg ctg tcg gcg gcg      1968
   Ser Phe Ile Phe Gly Lys Leu Asp Glu Gly Leu Ser Leu Ser Ala Ala
                   645                 650                 655
   acc ctg cag gcc cgg gcc aac ggc tac acc gag ccg gat ccg cgc gac      2016
   Thr Leu Gln Ala Arg Ala Asn Gly Tyr Thr Glu Pro Asp Pro Arg Asp
               660                 665                 670
   gat ctg tcc ggg atg gat gtg gcg cgt aag ctg ctg atc ctg gcg cgc      2064
   Asp Leu Ser Gly Met Asp Val Ala Arg Lys Leu Leu Ile Leu Ala Arg
           675                 680                 685
   gag gcc ggt tac aaa ctg gaa ctg agc gat atc gag gtc gag ccg gtg      2112
   Glu Ala Gly Tyr Lys Leu Glu Leu Ser Asp Ile Glu Val Glu Pro Val
       690                 695                 700
   ctg ccg ccg tcc ttc gac gcg tcg ggc gac gtg gac acc ttc ctg gcg      2160
   Leu Pro Pro Ser Phe Asp Ala Ser Gly Asp Val Asp Thr Phe Leu Ala
   705                 710                 715                 720
   cgg ttg ccg gag ctg gat aaa gag ttc gcg cgc aac gtg gct aac gcc      2208
   Arg Leu Pro Glu Leu Asp Lys Glu Phe Ala Arg Asn Val Ala Asn Ala
                   725                 730                 735
   gcc gag caa ggc aag gtg ctg cgc tat gtc ggc ctg atc gac gaa ggg      2256
   Ala Glu Gln Gly Lys Val Leu Arg Tyr Val Gly Leu Ile Asp Glu Gly
               740                 745                 750
   cgc tgc aag gtg cgc att gag gcg gtg gac ggc aac gat ccg ttg tat      2304
   Arg Cys Lys Val Arg Ile Glu Ala Val Asp Gly Asn Asp Pro Leu Tyr
           755                 760                 765
   aaa gtg aag aac ggc gag aac gcg ctg gcc ttc tac agc cgc tac tat      2352
   Lys Val Lys Asn Gly Glu Asn Ala Leu Ala Phe Tyr Ser Arg Tyr Tyr
       770                 775                 780
   cag ccg ttg ccg ctg gtg ctg cgc ggc tac ggc gcc ggt aac gat gtg      2400
   Gln Pro Leu Pro Leu Val Leu Arg Gly Tyr Gly Ala Gly Asn Asp Val
   785                 790                 795                 800
   act gca gcg ggc gta ttc gcc gat ctg ctg cgc aca ctg tca tgg aag      2448
   Thr Ala Ala Gly Val Phe Ala Asp Leu Leu Arg Thr Leu Ser Trp Lys
                   805                 810                 815
   ttg gga gtt taa                                                      2460
   Leu Gly Val
<210> 16
   <211> 819
   <212> PRT
   <213> Serratia marcescens
<400> 16
   Met Arg Val Leu Lys Phe Gly Gly Thr Ser Val Ala Asn Ala Glu Arg
   1               5                   10                  15
   Phe Leu Arg Val Ala Asp Ile Met Glu Ser Asn Ala Arg Gln Gly Gln
               20                  25                  30
   Val Ala Thr Val Leu Ser Ala Pro Ala Lys Ile Thr Asn His Leu Val
           35                  40                  45
   Ala Met Ile Asp Lys Thr Val Ala Gly Gln Asp Ile Leu Pro Asn Met
       50                  55                  60
   Ser Asp Ala Glu Arg Ile Phe Ala Asp Leu Leu Ser Gly Leu Ala Gln
   65                  70                  75                  80
   Ala Leu Pro Gly Phe Glu Tyr Asp Arg Leu Lys Gly Val Val Asp Gln
                   85                  90                  95
   Glu Phe Ala Gln Leu Lys Gln Val Leu His Gly Val Ser Leu Leu Gly
               100                 105                 110
   Gln Cys Pro Asp Ser Val Asn Ala Ala Ile Ile Cys Arg Gly Glu Lys
           115                 120                 125
   Leu Ser Ile Ala Ile Met Glu Gly Val Phe Arg Ala Lys Gly Tyr Pro
       130                 135                 140
   Val Thr Val Ile Asn Pro Val Glu Lys Leu Leu Ala Gln Gly His Tyr
   145                 150                 155                 160
   Leu Glu Ser Thr Val Asp Ile Ala Glu Ser Thr Leu Arg Ile Ala Ala
                   165                 170                 175
   Ala Ala Ile Pro Ala Asp His Ile Val Leu Met Ala Gly Phe Thr Ala
               180                 185                 190
   Gly Asn Asp Lys Gly Glu Leu Val Val Leu Gly Arg Asn Gly Ser Asp
           195                 200                 205
   Tyr Ser Ala Ala Val Leu Ala Ala Cys Leu Arg Ala Asp Cys Cys Glu
       210                 215                 220
   Ile Trp Thr Asp Val Asp Gly Val Tyr Thr Cys Asp Pro Arg Thr Val
   225                 230                 235                 240
   Pro Asp Ala Arg Leu Leu Lys Ser Met Ser Tyr Gln Glu Ala Met Glu
                   245                 250                 255
   Leu Ser Tyr Phe Gly Ala Lys Val Leu His Pro Arg Thr Ile Thr Pro
               260                 265                 270
   Ile Ala Gln Phe Gln Ile Pro Cys Leu Ile Lys Asn Thr Ser Asn Pro
           275                 280                 285
   Gln Ala Pro Gly Thr Leu Ile Gly Lys Asp Ser Thr Asp Ala Asp Met
       290                 295                 300
   Pro Val Lys Gly Ile Thr Asn Leu Asn Asn Met Ala Met Ile Asn Val
   305                 310                 315                 320
   Ser Gly Pro Gly Met Lys Gly Met Val Gly Met Ala Ala Arg Val Phe
   325 330 335
   Ala Val Met Ser Arg Ala Gly Ile Ser Val Val Leu Ile Thr Gln Ser
   340 345 350
   Ser Ser Glu Tyr Ser Ile Ser Phe Cys Val Pro Gln Gly Glu Leu Gln
   355 360 365
   Arg Ala Arg Arg Ala Leu Glu Glu Glu Phe Tyr Leu Glu Leu Lys Asp
   370 375 380
   Gly Val Leu Asp Pro Leu Asp Val Met Glu Arg Leu Ala Ile Ile Ser
   385 390 395 400
   Val Val Gly Asp Gly Met Arg Thr Leu Arg Gly Ile Ser Ala Arg Phe
   405 410 415
   Phe Ser Ala Leu Ala Arg Ala Asn Ile Asn Ile Val Ala Ile Ala Gln
   420 425 430
   Gly Ser Ser Glu Arg Ser Ile Ser Val Val Val Ser Asn Asp Ser Ala
   435 440 445
   Thr Thr Gly Val Arg Val Ser His Gln Met Leu Phe Asn Thr Asp Gln
   450 455 460
   Val Ile Glu Val Phe Val Ile Gly Val Gly Gly Val Gly Gly Ala Leu
   465 470 475 480
   Ile Glu Gln Ile Tyr Arg Gln Gln Pro Trp Leu Lys Gln Lys His Ile
   485 490 495
   Asp Leu Arg Val Cys Gly Ile Ala Asn Ser Arg Val Met Leu Thr Asn
   500 505 510
   Val His Gly Ile Ala Leu Asp Ser Trp Arg Asp Ala Leu Ala Gly Ala
   515 520 525
   Gln Glu Pro Phe Asn Leu Gly Arg Leu Ile Arg Leu Val Lys Glu Tyr
   530 535 540
   His Leu Leu Asn Pro Val Ile Val Asp Cys Thr Ser Ser Gln Ala Val
   545 550 555 560
   Ala Asp Gln Tyr Val Asp Phe Leu Ala Asp Gly Phe His Val Val Thr
   565 570 575
   Pro Asn Lys Lys Ala Asn Thr Ser Ser Met Asn Tyr Tyr Gln Gln Leu
               580                 585                 590
   Arg Ala Ala Ala Ala Gly Ser His Arg Lys Phe Leu Tyr Asp Thr Asn
           595                 600                 605
   Val Gly Ala Gly Leu Pro Val Ile Glu Asn Leu Gln Asn Leu Leu Asn
       610                 615                 620
   Ala Gly Asp Glu Leu Val Arg Phe Ser Gly Ile Leu Ser Gly Ser Leu
   625                 630                 635                 640
   Ser Phe Ile Phe Gly Lys Leu Asp Glu Gly Leu Ser Leu Ser Ala Ala
                   645                 650                 655
   Thr Leu Gln Ala Arg Ala Asn Gly Tyr Thr Glu Pro Asp Pro Arg Asp
               660                 665                 670
   Asp Leu Ser Gly Met Asp Val Ala Arg Lys Leu Leu Ile Leu Ala Arg
           675                 680                 685
   Glu Ala Gly Tyr Lys Leu Glu Leu Ser Asp Ile Glu Val Glu Pro Val
       690                 695                 700
   Leu Pro Pro Ser Phe Asp Ala Ser Gly Asp Val Asp Thr Phe Leu Ala
   705                 710                 715                 720
   Arg Leu Pro Glu Leu Asp Lys Glu Phe Ala Arg Asn Val Ala Asn Ala
                   725                 730                 735
   Ala Glu Gln Gly Lys Val Leu Arg Tyr Val Gly Leu Ile Asp Glu Gly
               740                 745                 750
   Arg Cys Lys Val Arg Ile Glu Ala Val Asp Gly Asn Asp Pro Leu Tyr
           755                 760                 765
   Lys Val Lys Asn Gly Glu Asn Ala Leu Ala Phe Tyr Ser Arg Tyr Tyr
       770                 775                 780
   Gln Pro Leu Pro Leu Val Leu Arg Gly Tyr Gly Ala Gly Asn Asp Val
   785                 790                 795                 800
   Thr Ala Ala Gly Val Phe Ala Asp Leu Leu Arg Thr Leu Ser Trp Lys
                   805                 810                 815
   Leu Gly Val
<210> 17
   <211> 42
   <212> PRT
   <213> Escherichia coli
<220>
   <221> POLYPEPTIDE
   <222> (1)..(92)
   <223> Xaa = beliebige Aminosäure
<400> 17
   Pro Gly Xaa Leu Phe Val His Arg Asn Val Ala Asn Leu Val Ile His
   1               5                   10                  15
   Thr Asp Leu Asn Cys Leu Ser Val Xaa Gln Tyr Ala Val Xaa Val Leu
               20                  25                  30
   Glu Val Glu His Ile Ile Ile Cys Gly His
           35                  40
<210> 18
   <211> 22
   <212> PRT
   <213> Escherichia coli
<220>
   <221> POLYPEPTIDE
   <222> (1)..(22)
   <223> Xaa = beliebige Aminosäure
<400> 18
   Thr Gln Xaa Glu Pro Gly Xaa Leu Phe Val Ile Arg Asn Ala Gly Asn
   1               5                   10                  15
   Ile Val Pro Ser Xaa Gly
               20

## Beschreibung

Diese Erfindung betrifft ein Verfahren zur fermentativen Herstellung von L-Threonin und L-Tryptophan unter Verwendung von rekombinanten Mikroorganismen der Familie Enterobacteriaceae, in denen mindestens eines oder mehrere der Gene, ausgewählt aus der Gruppe can und cynT, welche für Carboanhydrasen kodieren, verstärkt, insbesondere überexprimiert wird/werden, und diese Mikroorganismen.

### Stand der Technik

L-Aminosäuren, insbesondere L-Threonin, L-Tryptophan, L-Lysin, L-Valin, L-Isoleucin und L-Homoserin, finden in der Humanmedizin und in der pharmazeutischen Industrie, in der Lebensmittelindustrie und in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht Aminosäure überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren. Resistenz gegen das Tryptophan-Analogon 5-Methyl-DL-Tryptophan (5-MT) zeichnet zum Beispiel einen Stamm aus, der L-Tryptophan produziert.

Seit einigen Jahren werden ebenfalls Methoden der rekombinanten DNA-Technik zur gezielten Stammverbesserung L-Aminosäuren produzierender Stämme der Familie Enterobacteriaceae eingesetzt, indem man zum Beispiel einzelne Aminosäure-Biosynthesegene amplifiziert oder die Eigenschaften spezieller Gene verändert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)).

Die Gene can (früher: yadF) und cynT kodieren für Carboanhydrasen, diese Enzyme katalysieren die reversible Hydrierung von CO2 zum Bicarbonat-Anion (Merlin et al., Journal of Bacteriology 185(21): 6415-6424 (2003)). Das Gleichgewicht dieser Reaktion liegt bei pH 6.3; bei höheren pH-Werten überwiegt das HCO3⁻, bei niedrigeren das CO2. Um die intrazellulär benötigten CO2/HCO3- -Konzentrationen bei physiologischem pH aufrecht zu erhalten, kommt der Carboanhydrase im Kohlenstoff-Metabolismus und in der Modulation des Säure-Basen-Gleichgewichts durch die CO2-Fixierung bzw. Bereitstellung eine Schlüsselfunktion zu.

Das cynTSX-Operon, kodierend für eine Cyanat-Hydratase (cynS), eine Carboanhydrase (cynT) und ein Protein unbekannter Funktion (cynX), ermöglicht es Escherichia coli, auf Cyanat als einziger Stickstoffquelle zu wachsen (Sung und Fuchs, Journal of Biological Chemistry 263: 14769-14775 (1988)). Durch die Cyanat-Hydratase wird Cyanat mit Bicarbonat zu Ammonium und Kohlendioxid umgesetzt und die Carboanhydrase spielt zur Re-Fixierung des freigesetzten Kohlendioxids eine wichtige Rolle, cynT - Mutanten werden in ihrem Wachstum auf Cyanat stark gehemmt (Guilloton et al., Journal of Bacteriology 175(5): 1443-1451 (1993)).

Die Carboanhydrase YadF spielt eine essentielle Rolle in der Zellvermehrung (Hashimoto und Kato, Bioscience, Biotechnology, and Biochemistry 67(4): 919-922 (2003)), da HCO3- für diverse Bicarbonat-abhängige Reaktionen/Enzyme, wie zum Beispiel für Phosphoenolpyruvat oder Acetyl-CoA-Carboxylase (Smith und Ferry, FEMS 24:335-366 (1999)), bereitgestellt wird.

Can und CynT gehören zur β-Klasse der Carboanhydrasen und die Induktion der Expression des cynT-Gens supprimiert den Phänotyp einer can- Mutante (Merlin et al., Journal of Bacteriology 185(21): 6415-6424 (2003)). Eine can-/cynT-Doppelmutante ist nur unter hohen CO2-Konzentrationen lebensfähig (Hashimoto und Kato, Bioscience, Biotechnology, and Biochemistry 67(4): 919-922 (2003)).

Die Nukleotidsequenzen der Wildformen der für die Carboanhydrasen von Escherichia coli kodierenden Gene ist in der Datenbank des National Center for Biotechnology Information (NCBI) der National Library of Medicin (Bethesda, MD, USA) unter den Zugangsnummern (accession number) NC_000913 (Region: 142008 <- 142670 (can)) und NC_000913 (Region: 358023 -> 358682 (cynT)) allgemein verfügbar,

### Aufgabe der Erfindung

Die Erfinder haben sich die Aufgabe gestellt, neue Maßnahmen zur Herstellung erhöhter Mengen von L-Threonin und L-Tryptophan bereitzustellen.

### Beschreibung der Erfindung

Gegenstand der Erfindung sind rekombinante L-Threonin oder L-Tryptophan produzierende Mikroorganismen der Familie Enterobacteriaceae, die ein verstärktes oder überexprimiertes can-Gen und/oder cynT-Gen enthalten, welche für Carboanhydrasen kodieren, wobei die L-Threonin produzierenden Mikroorganismen zusätzlich eines oder mehrere der Gene, ausgewählt aus der Gruppe:
- mindestens ein Gen des für die Aspartatkinase, die Homoserin-Dehydrogenase, die Homoserinkinase und die Threoninsynthase kodierenden thrABC-Operons (US-A-4, 278, 765) ,
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992); WO 97/08333),
- das für die Phosphoenolpyruvat-Carboxylase kodierende ppc-Gen (WO 02/064808),
- die für die Untereinheiten der Pyridin-Transhydrogenase kodierenden Gene pntA und pntB (European Journal of Biochemistry 158: 647-653 (1986); WO 95/11985),
- das Gen rhtC, welches für das Threoninresistenz vermittelnde Protein kodiert
   (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983); DE19907347),
- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glucose-spezifische IIA Komponente des Phosphotransferase-Systems PTS kodierende crr-Gen des ptsHIcrr-Operons (WO 03/004674),
- das für die Glucose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Reduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD--Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615),
- das Genprodukt des offenen Leserahmens (ORF) yjcG von Escherichia coli (Accession Number NC000913 (Region 4281276-4282925) des National Center for Biotechnology Information (NCBI, Bethesda, MD, USA),
- das Genprodukt des offenen Leserahmens (ORF) yibD von Escherichia coli (Accession Number AE000439 des National Center for Biotechnology Information (NCBI, Bethesda, MD, USA)),
- das Genprodukt des offenen Leserahmens (ORF) yaaU von Escherichia coli (Accession Number NC000913 (Region 45807-47138) des National Center for Biotechnology Information (NCBI, Bethesda, MD, USA), der auch unter der Bezeichnung yaaU-ORF bekannt ist,
- das für das universelle Stressprotein UspC kodierende uspC-Gen (Accession Number NC000913 (Region 1977777-1978205) des National Center for Biotechnology Information (NCBI, Bethesda, MD, USA)),
- das uspD-Gen, welches für das Resistenz gegen UV-Strahlung vermittelnde universelle Stressprotein UspD kodiert (Accession Number NC000913 (Region 4111316-4111744) des National Center for Biotechnology Information (NCBI, Bethesda, MD, USA)),
- das uspE-Gen, welches für das Resistenz gegen UV-Strahlung vermittelnde universelle Stressprotein UspE kodiert (Accession Number NC000913 (Region 1395696-1396646) des National Center for Biotechnology Information (NCBI, Bethesda, MD, USA)),
   verstärkt oder überexprimiert enthalten, und die L-Tryptophan produzierenden Mikroorganismen zusätzlich gleichzeitig eines oder mehrere der Gene, ausgewählt aus der Gruppe:
- mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons (Applied and Environmental Microbiology 38(2): 181-190 (1979)),
- das für die 3-Phosphoglycerat-Dehydrogenase kodierende serA-Gen (WO87/01130)
- das für die Phosphoserinphosphatase kodierende serB-Gen (WO87/01130)
- das für die Phosphoserinaminotransferase kodierende serC-Gen (WO87/01130)
- das für die L-Tyrosin sensitive DHAP-Synthase kodierende aroF-Gen (WO87/011 EP1270721)
- das für die L-Phenylalanin sensitive DHAP-Synthase kodierende aroG-Gen (WO87/01130; EP1270721)
- das für die L-Tryptophan sensitive DHAP-Synthase kodierende aroH-Gen (WO87/011 EP1270721)
- das für die Phosphoenolpyruvat-Synthase kodierende pps-Gen (WO96/08567)
- das für die Phosphoenolpyruvat-Carboxykinase kodierende pckA-Gen (WO2004/090125),
- das für die Transketolase A kodierende tktA-Gen (US5,168,056),
- das für die Transketolase B kodierende tktB-Gen (US5,168,056),
- das Genprodukt des offenen Leserahmens (ORF) yddG von Escherichia coli (Accession Number NCO00913 (Region 1544312-1545193) des National Center for Biotechnology Information (NCBI, Bethesda, MD, USA)),
- das für das universelle Stressprotein UspC kodierende uspC-Gen (Accession Number NC000913 (Region 1977777-1978205) des National Center for Biotechnology Information (NCBI, Bethesda, MD, USA)),
- das für das Resistenz gegen UV-Strahlung vermittelnde universelle Stressprotein UspD kodierende uspD-Gen (Accession Number NC000913 (Region 4111316-4111744) des National Center for Biotechnology Information (NCBI, Bethesda, MD, USA)),
- das für das Resistenz gegen UV-Strahlung vermittelnde universelle Stressprotein UspE kodierende uspE-Gen (Accession Number NC000913 (Region 1395696-1396646) des National Center for Biotechnology Information (NCBI, Bethesda, MD, USA)),

### verstärkt oder überexprimiert enthalten.

Die erfindungsgemäßen Mikroorganismen produzieren vorzugsweise vermehrt L-Threonin oder L-Tryptophan und reichern das L-Threonin oder L-Tryptophan in der Zelle oder im Medium an.

Als Ausgangspunkt für den Vergleich dienen jeweils die für die Gene can und cynT nicht rekombinanten Mikroorganismen, die kein verstärktes can- bzw. cynT-Gen enthalten und an denen die erfindungsgemäße Verstärkung oder Überexpression nicht durchgeführt wurde.

Zu den erfindungsgemäßen Mikroorganismen gehören insbesondere Mikroorganismen der Familie Enterobacteriaceae, in denen
a) ein als can-Gen bezeichnetes Polynukleotid, das für ein Polypeptid kodiert, dessen Aminosäuresequenz zu mindestens 80% oder zu mindestens 90%, insbesondere zu mindestens 95%, bevorzugt zu mindestens 98% oder mindestens 99%, besonders bevorzugt zu 99,6% und ganz besonders bevorzugt zu 100% identisch ist mit einer Aminosäuresequenz, ausgewählt aus der Gruppe SEQ ID No. 2, SEQ ID No. 4 und SEQ ID No. 6, bevorzugt SEQ ID No. 2, und
b) ein als cynT-Gen bezeichnetes Polynukleotid, das für ein Polypeptid kodiert, dessen Aminosäuresequenz zu mindestens 80% oder zu mindestens 90%, insbesondere zu mindestens 95%, bevorzugt zu mindestens 98% oder mindestens 99%, besonders bevorzugt zu 99,6% und ganz besonders bevorzugt zu 100% identisch ist mit einer Aminosäuresequenz, ausgewählt aus der Gruppe SEQ ID No. 8, SEQ ID No. 10 und SEQ ID No. 12, bevorzugt SEQ ID No. 8,
   einzeln oder gemeinsam verstärkt werden (wird), wobei die Polypeptide die Aktivität einer Carboanhydrase besitzt.

Zu den erfindungsgemäßen Mikroorganismen gehören ebenso insbesondere Mikroorganismen der Familie Enterobacteriaceae, in denen einzeln oder gemeinsam als can- und cynT-Gen bezeichnete Polynukleotide verstärkt werden, wobei
a) das can-Gen für ein Polypeptid kodiert, dessen Aminosäuresequenz zwischen den Positionen 57 und 98 oder vergleichbaren Positionen, die Sequenz ProGlyXaaLeuPheValHisArgAsnValAlaAsnLeuValIleHisThrAsp LeuAsnCysLeuSerValXaaGlnTyrAlaValXaaValLeuGluValGluHis IleIleIleCysGlyHis (SEQ ID No. 17) enthält, , wobei an Position 1 der Aminosäuresequenz das Methionin kodiert durch das Startcodon AUG oder GUG steht und Xaa eine beliebige Aminosäure sein kann, und das im Wesentlichen eine Länge von 220 Aminosäuren umfasst,
   und
b) das cynT-Gen für ein Polypeptid kodiert, dessen Aminosäuresequenz zwischen Position 50 und 71 oder vergleichbaren Positionen, die Sequenz ThrGlnXaaGluProGlyXaaLeuPheValIleArgAsnAlaGlyAsnIleVal ProSerXaaGly (SEQ ID No. 18) enthält, wobei an Position 1 der Aminosäuresequenz das Methionin kodiert durch das Startcodon AUG oder GUG steht und Xaa eine beliebige Aminosäure sein kann,
und das im Wesentlichen eine Länge von 219 Aminosäuren umfasst, wobei das Polypeptid die Aktivität einer Carboanhydrase besitzt.

Vergleichbare Positionen lassen sich durch Vergleich der Aminosäuresequenzen in Form eines "alignments" beispielsweise mit Hilfe des Clustal W-Programmes (Thompson et al., Nucleic Acids Research 22, 4637-4680 (1994)) oder mit Hilfe des MAFFT-Programmes (Katoh et al., Genome Information 2005; 16(1),22-33) leicht identifizieren.

Die erfindungsgemäßen Mikroorganismen enthalten bevorzugt als can-Gen bezeichnete, verstärkte isolierte Polynukleotide, ausgewählt aus der Gruppe:
a) Polynukleotid mit einer Nukleotidsequenz, ausgewählt aus SEQ ID No. 1, SEQ ID No. 3, SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1, SEQ ID No. 3 oder SEQ ID No. 5 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält, und/oder
   bevorzugt als cynT-Gen bezeichnete, verstärkte isolierte Polynukleotide, ausgewählt aus der Gruppe:
e) Polynukleotid mit einer Nukleotidsequenz, ausgewählt aus SEQ ID No. 7, SEQ ID No. 9, SEQ ID No. 11 und den dazu komplementären Nukleotidsequenzen;
f) Polynukleotid mit einer Nukleotidsequenz, die der SEQ ID No. 7, SEQ ID No. 9 oder SEQ ID No. 11 im Rahmen der Degeneration des genetischen Codes entspricht;
g) Polynukleotidsequenz mit einer Sequenz, die mit der zur Sequenz SEQ ID No. 7, SEQ ID No. 9 oder SEQ ID No. 11 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
h) Polynukleotid mit einer Sequenz SEQ ID No. 7, SEQ ID No. 9 oder SEQ ID No. 11, die funktionsneutrale Sinnmutanten enthält,
wobei die Polynukleotide für eine Carboanhydrase kodieren.

Die erfindungsgemäßen rekombinanten Mikroorganismen der Familie Enterobacteriaceae produzieren vorzugsweise bereits vor der erfindungsgemäßen Verstärkung oder Überexpression L-Aminosäuren.

Als vor der erfindungsgemäßen Verstärkung oder Überexpression L-Aminosäure produzierende Mikroorganismen zählen dabei nicht die Wildtypstämme und häufig benutzte Laborstämme wie unter anderen DH5α, DH5αmcr, W3110, JM105, MG1655, MC4100, Y1089, H560 und MM152.

Bevorzugt werden die beschriebenen Mikroorganismen eingesetzt.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin gemeint. Besonders bevorzugt sind L-Threonin, L-Tryptophan, L-Lysin, L-Valin, L-Isoleucin und L-Homoserin.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Verstärkung oder Überexpression durchgeführt wird.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Gebräuchliche Methoden zur Präparation der Proteingele und zur Identifizierung der Proteine sind die im 2-D Electrophoresis Handbook (GE Healthcare UK Ltd, England, Handbook 80-6429-60AC (2006)) und die von Hermann et al. (Electrophoresis, 22:1712-23 (2001))beschriebenen Vorgehensweisen. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden.

Ein Gen oder Allel ist chemisch ein Polynukleotid. Ein anderer Begriff hierfür ist Nukleinsäure, insbesondere Desoxyribonukleinsäure.

Die Begriff Polypeptid und Protein sind gegenseitig austauschbar.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Gegenstand der Erfindung ist ein Verfahren zur Herstellung von L-Aminosäuren durch Fermentation der erfindungsgemäßen rekombinanten Mikroorganismen der Familie Enterobacteriaceae, dadurch gekennzeichnet, dass man
a) die die gewünschte L-Aminosäure produzierenden Mikroorganismen in einem Medium kultiviert unter Bedingungen, bei denen die gewünschte L-Aminosäure im Medium oder in den Zellen angereichert wird, und,
b) die gewünschte L-Aminosäure isoliert, wobei Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die erfindungsgemäßen rekombinanten Mikroorganismen können L-Aminosäuren aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens oder ORFs verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Zur Herstellung der L-Aminosäure-anreichernden Stämme der Familie Enterbacteriaceae, welche ein verstärktes can- und/oder cynT-Gen enthalten, werden bevorzugt Stämme verwendet (Ausgangsstämme bzw. Elternstämme), die bereits die Fähigkeit besitzen, die gewünschte L-Aminosäure in der Zelle anzureichern und/oder in das sie umgebende Nährmedium auszuscheiden bzw. in der Fermentationsbrühe zu akkumulieren. Hierfür wird in diesem Zusammenhang auch der Ausdruck "produzieren" verwendet. Insbesondere besitzen die für die Maßnahmen der Erfindung eingesetzten Stämme die Fähigkeit ≥ (mindestens) 0,25 g/l, ≥ 0,5 g/l, ≥1,0 g/l, ≥ 1,5 g/l, ≥ 2,0 g/l, ≥ 4 g/l oder ≥ 10 g/l an L-Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle und/oder im Nährmedium bzw. der Fermentationsbrühe anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Die genannten L-Aminosäuren-anreichernden Stämme produzieren eine oder mehrere, bevorzugt eine oder im Wesentlichen eine Aminosäure ausgewählt aus L-Threonin und L-Tryptophan. Der Begriff L-Aminosäure oder Aminosäure umfasst auch deren Salze.

Der Begriff " eine oder im Wesentlichen eine Aminosäure" berücksichtigt, dass zusätzlich zu der gewünschten L-Aminosäure eine oder mehrer weitere der genannten L-Aminosäuren (Nebenaminosäuren) produziert werden können. Der Anteil dieser Nebenaminosäuren beträgt ≥ 0 bis maximal 40%, bevorzugt ≥ 0 bis maximal 20%, besonders bevorzugt ≥ 0 bis maximal 10% und ganz besonders bevorzugt ≥ 0 bis maximal 5% bezogen auf die Menge der gewünschten L-Aminosäure.

Als zum Elternstamm geeignete, insbesondere L-Threonin produzierende bzw. ausscheidende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli TH 14.97 (WO 02/26993)
- Escherichia coli TH 21.97 (WO 02/26993)
- Escherichia coli BKIIM B-3996 (US-A-5,175,107)
- Escherichia coli BKIIM B-3996ΔtdhΔpckA/pVIC40 (WO 02/29080)
- Escherichia coli kat 13 (WO 98/04715)
- Escherichia coli Kat 69.9 (WO 02/26993)
- Escherichia coli KCCM-10132 (WO 00/09660)
- Escherichia coli KCCM-10168 (WO 01/14525)
- Escherichia coli KCCM-10133 (WO 00/09661)

Als zum Elternstamm geeignete L-Threonin produzierende bzw. ausscheidende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992)) L-Threonin produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Als zum Elternstamm geeignete, L-Tryptophan produzierende bzw. ausscheidende Stämme der Gattung Escherichia, insbesondere der Art Escherichia coli sind beispielsweise zu nennen:
- Escherichia coli JP4735/pMU3028 (US5,756,345)
- Escherichia coli JP6015/pMU91 (US5,756,345)
- Escherichia coli SV164(pGH5) (WO94/08031)
- E. coli AGX17 (pGX44) (NRRL B-12263) (US4, 371, 614)
- E. coli AGX6(pGX50)aroP (NRRL B-12264) (US4,371,614)
- Escherichia coli AGX17/pGX50,pACKG4-pps (WO97/08333)
- Escherichia coli ATCC 31743 (CA1182409)
- E. coli C534/pD2310,pDM136 (ATCC 39795) (WO87/01130)
- Escherichia coli JB102/p5LRPS2 (US5,939,295).

L-Tryptophan produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phophoenolpyruvat-Synthese, Verstärkung der D-Erythrose-4-Phosphat-Synthese, Verstärkung der 3-deoxy-D-arabino-heptulosonat-7-Phosphat(DHAP)-Synthese, Verstärkung der Chorismat-Biosynthese.

Bei den der Erfindung zugrunde liegenden Arbeiten wurde gefunden, dass Mikroorganismen der Familie Enterobacteriaceae nach Überexpression des Gens can und/oder des Gens cynT, oder deren Allele, vermehrt L-Threonin und L-Tryptophan produzieren und in der Zelle oder im Medium anreichern.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminopeptidase) die N-terminale Aminosäure Methionin abgespalten werden kann.

Aus den ebenfalls zur Familie Enterobacteriaceae gehörenden Salmonella enterica und Photorhabdus luminescens ist die Nukleotidsequenz für das can(yadF)-Gen ebenso bekannt (Accession No.: NC_006511 (REGION: 203687-204349 komplementär) und Accession No.: NC_005126 (REGION: 995109-995762)). Weitere Nukleotidsequenzen für das can-Gen findet man aus folgenden Enterobacteriaceae: Shigella dysenteriae (Accession No.: NC_007606); Shigella boydii (Accession No.: NC_007613); Enterobacter (Accession No.: NC_009436, NC_009778); Klebsiella pneumoniae (Accession No.: NC_009648); Erwinia carotovora (Accession No.: NC_004547); Citrobacter koseri (Accession No.: NC_009792); Salmonella typhimurium (Accession No.: NC_003197); Sodalis glossinidius (Accession No.: NC_007712); Serratia proteamaculans (Accession No.: NC_009832).

Aus den ebenfalls zur Familie Enterobacteriaceae gehörenden Serratia proteamaculans und Photorhabdus luminescens ist die Nukleotidsequenz für das cynT-Gen ebenso bekannt (Accession No.: NC_009832 (Region: 1671188-1671841)) und Accession No.: NC_005126 (REGION: 114687-115346)). Weitere Nukleotidsequenzen für das cynT-Gen findet man aus folgenden Enterobacteriaceae: Enterobacter (Accession No.: NC_009436); Klebsiella pneumoniae (Accession No.: NC_009648); Pseudomonas putida (Accession No.: NZ_AAVY01000002); Burkholderia pseudomallei (Accession No.: NC_009076).

Das can-Gen von Escherichia coli K12 wird unter anderem durch folgende Angaben beschrieben:

| | |
|---|---|
| Bezeichnung: | Carboanhydrase 2, Can |
| Funktion: | das pH-abhängige Enzym katalysiert die reversible Hydrierung von CO2 zum Bicarbonat-Anion; essentielle Rolle in der Zellvermehrung durch Bereitstellung von HCO3- für diverse Bicarbonat-abhängige Reaktionen/Enzyme |
| Referenz: | Merlin et al., Journal of Bacteriology 185(21): 6415-6424 (2003) Cronk et al., Protein Science 10(5);911-22 (2001) |
| | Smith und Ferry, FEMS 24:335-366 (1999) Tripp et al., Journal of Biological Chemistry 276(52): 48615-48618 (2001) Hashimoto und Kato, Bioscience, Biotechnology, and Biochemistry 67(4): 919-922 (2003) |
| Accession No.: | NC_000913 (REGION: 142008 <- 142670) |

Das kodierte Polypeptid besitzt eine Länge von 220 Aminosäuren.

Alternativer Genname: B0126 , YadF

Das cynT-Gen von Escherichia coli K12 wird unter anderem durch folgende Angaben beschrieben:

| | |
|---|---|
| Bezeichnung: | Carboanhydrase 1, CynT |
| Funktion: | das Enzym katalysiert die reversible Hydrierung von CO2 zum Bicarbonat-Anion; ein Gen des cynTSX-Operons, welches es Escherichia coli |
| | ermöglicht, auf Cyanat als einziger Stickstoffquelle zu wachsen |
| Referenz: | Merlin et al., Journal of Bacteriology 185 (21) : 6415-6424 (2003) Guilloton et al., Journal of Bacteriology |
| | 175(5): 1443-1451 (1993) Sung und Fuchs, Journal of Biological Chemistry 263: 14769-14775 (1988) |
| Accession No.: | NC_000913 (REGION: 358023 -> 358682) |

Das kodierte Polypeptid besitzt eine Länge von 219 Aminosäuren.

Alternativer Genname: B0339

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum can-Gen von Escherichia coli unter der SEQ ID No. 1 und die bekannten Sequenzen zum can-Gen von Salmonella enterica und Photorhabdus luminescens unter den SEQ ID No. 3 und SEQ ID No. 5 dargestellt. Die von diesen Leserahmen kodierten Proteine sind als SEQ ID No. 2, SEQ ID No. 4 und SEQ ID No. 6 dargestellt.

Desweiteren sind die bekannte Sequenz zum cynT-Gen von Escherichia coli unter der SEQ ID No. 7 und die bekannten Sequenzen zum cynT-Gen von Serratia proteamaculans und Photorhabdus luminescens unter den SEQ ID No. 9 und SEQ ID No. 11 dargestellt. Die von diesen Leserahmen kodierten Proteine sind als SEQ ID No. 8, SEQ ID No. 10 und SEQ ID No. 12 dargestellt.

Die in den angegebenen Textstellen beschriebenen Gene oder offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des Gene can bzw. cynT, welche funktionsneutrale Sinnmutationen enthalten, zählen unter anderem solche, die zu höchstens '15, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 8 in keinem Falle aber mehr als 10 Aminosäuren betreffen.

Zu den geeigneten Allelen gehören ferner solche, die durch Hybridisierung, insbesondere unter stringenten Bedingungen unter Verwendung von SEQ ID No. 1, SEQ ID No. 3 oder SEQ ID No. 5 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 75% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 75% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 75% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1, SEQ ID No. 3 oder SEQ ID No. 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Zur Erzielung einer Verstärkung, insbesondere einer Überexpression, können beispielsweise die Expression der Gene oder offenen Leserahmen oder Allele oder die katalytischen Eigenschaften des Proteins erhöht werden. Gegebenenfalls können beide Maßnahmen kombiniert werden.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens, bevorzugt des uspA-Gens, befindet, mutiert werden. In gleicher Weise wirken Expressionskassetten oder Promotoren, die stromaufwärts des Strukturgens eingebaut werden. Durch induzierbare Promotoren ist es zusätzlich möglich, die Expression im Verlaufe der fermentativen L-Aminosäure-Produktion zu steigern. Des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene, ORFs oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)), die Verwendung dieser Plasmide ohne Induktion durch IPTG-Zugabe (= Isopropyl-β-D-Thiogalactopyranosid) führt zu einer sehr schwachen Expression von ohne eigenem Promotor klonierten Genen. Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Der Austausch des Promotors gegen einen zum Beispiel in genomweit-vergleichenden Expressionsanalysen ermittelten Promotor mit gleichmäßig hoher Expression im Verlauf eines Fermentationsprozesses bewirkt eine gleichmäßige Verstärkung. Die Veränderung einer Wachstumsphasen-abhängigen Genexpression kann beispielsweise wie bei Walker et al. (Journal of Bacteriology 181: 1269-80 (1999)) beschrieben durch die Verwendung des Wachstumsphasen-abhängigen fis Promotors oder einer der dort beschriebenen Mutationen des fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden. Desweiteren kann der Austausch eines Start-Codons zu dem in Escherichia coli mit 77% am häufigsten vorkommenden Codon ATG die Translation erheblich verbessern, da das Codon AUG zwei- bis dreimal effektiver ist als zum Beispiel die Codons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Codons kann optimiert werden, da zusammenwirkende Effekte zwischen dem Start-Codon und den flankierenden Bereichen beschrieben sind (Stenstrom et al., Gene 273(2):259-65 (2001); Hui et al., EMBO Journal 3(3):623-9 (1984)).

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolomé et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens das can-Gen und/oder das cynT-Gen, oder für dessen Genprodukt kodierende Nukleotidsequenzen oder Allele trägt.

Unter dem Begriff Transformation versteht man die Aufnahme einer isolierten Nukleinsäure durch einen Wirt (Mikroorganismus).

Zur Erzeugung von can- oder cynT-Allelen, die in den für das erfindungsgemäße Verfahren eingesetzten Mikroorganismen zum Einsatz kommen, können unter anderem im Stand der Technik beschriebene Methoden zur gerichteten Mutagenese verwendet werden.

Es können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Zur Konstruktion von Punktmutationen im can-Gen und/oder cynT-Gen kann zum Beispiel das Quick Change Site-Directed Mutagenesis Kit der Firma Stratagene (Amsterdam, Niederlande) verwendet werden. Bei Verwendung dieser Methoden wird das im Stand der Technik beschriebene uspA-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden.

Die erzeugten can- oder cynT-Allele können beispielsweise durch Transformation und das Verfahren des Gen- bzw. Allelaustausches in geeignete Stämme eingebaut werden.

Eine gebräuchliche Methode ist die von Hamilton et al. (Journal of Bacteriology 171, 4617 - 4622 (1989)) beschriebene Methode des Genaustauschs mit Hilfe eines konditional replizierenden pSC101.-Derivates pMAK705 oder mit pKO3 (Link et al., Journal of Bacteriology 179 : 6228-6237 (1997)). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Es ist ebenfalls möglich die erzeugten can- oder cynT-Allele durch Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001). L-Threonin produzierende Mikroorganismen der Familie Enterobacteriaceae besitzen typischerweise eine "feed back" resistente bzw. desensibilisierte Aspartatkinase I/Homoserin-Dehydrogenase I. Unter "feed back" resistenter Aspartatkinase/Homoserin-Dehydrogenase versteht man Aspartatkinase/Homoserin-Dehydrogenase Enzyme (kodiert durch thrA, EC:2.7.2.4/ EC:1.1.1.3), die im Vergleich zur Wildform eine geringere Empfindlichkeit gegenüber der Hemmung durch Threonin oder Mischungen von Threonin und dem Threonin-Analogon α-Amino-β-Hydroxyvaleriansäure (AHV) oder AHV allein aufweisen. Die für diese desensibilisierten Enzyme kodierenden Gene bzw. Allele werden auch als thrA^{FBR}-Allele bezeichnet. Im Stand der Technik sind thrA^{FBR}-Allele beschrieben, die für Aspartatkinase/Homoserin-Dehydrogenase Varianten kodieren, welche Aminosäureaustausche im Vergleich zum Wildtypprotein besitzen. Die Kodierregion des Wildtyp thrA-Gens von Escherichia coli entsprechend der Zugangsnummer U00096.2 der NCBI Datenbank (Bethesda, MD, USA) ist in SEQ ID No.13 und das von diesem Gen kodierte Polypeptid in SEQ ID No.14 dargestellt.

Auch die Nukleotidsequenz des thrA-Gens von Serratia marcescens ist bekannt und unter der Zugangsnummer X60821 beim NCBI verfügbar. Die Kodierregion des Wildtyp thrA-Gens von Serratia marcescens ist in SEQ ID No.15 und das von diesem Gen kodierte Polypeptid in SEQ ID No.16 dargestellt.

Die für die Maßnahmen der Erfindung eingesetzten L-Threonin produzierenden Mikroorganismen der Familie Enterobacteriaceae verfügen bevorzugt über ein thrA-Allel, das für eine Aspartatkinase/Homoserin-Dehydrogenase Variante kodiert, welche die Aminosäuresequenz von SEQ ID No.14 oder SEQ ID No.16 besitzt, wobei diese eine oder mehrere der Aminosäureaustausche ausgewählt aus der Gruppe:
ThrA E253K (Austausch von L-Glutaminsäure an Position 253 des kodierten Enzyms Aspartatkinase/Homoserin-Dehydrogenase gemäß SEQ ID No.14 oder SEQ ID No.16 gegen L-Lysin; siehe Research Disclosure 505, 537 (2006)),
ThrA G330D (Austausch von Glycin an Position 330 des kodierten Enzyms Aspartatkinase/Homoserin-Dehydrogenase gemäß SEQ ID No.14 oder SEQ ID No.16 gegen L-Asparaginsäure; siehe Omori et al. (Journal of Bacteriology 175(3), 785-794 (1993)),
ThrA S345F (Austausch von L-Serin an Position 345 des kodierten Enzyms Aspartatkinase/Homoserin-Dehydrogenase gemäß SEQ ID No.14 oder SEQ ID No.16 gegen L-Phenylalanin; siehe Lee et al., Journal of Bacteriology 185(18): 5442-5451 (2003)),
ThrA S352, Austausch von L-Serin an Position 352 des kodierten Enzyms Aspartatkinase/Homoserin-Dehydrogenase gemäß SEQ ID No.14 oder SEQ ID No.16 gegen L-Phenylalanin, L-Tyrosin, L-Asparagin, L-Alanin, L-Arginin, L-Glutamin, L-Glutaminsäure, L-Histidin, L-Leucin, L-Methionin, L-Tryptophan oder L-Valin, bevorzugt gegen L-Phenylalanin; siehe Omori et al. (Journal of Bacteriology 175(3), 785-794 (1993) und Omori et al. (Journal of Bacteriology 175(4), 959-965 (1993),
ThrA A479T (Austausch von L-Alanin an Position 479 des kodierten Enzyms Aspartatkinase/Homoserin-Dehydrogenase gemäß SEQ ID No.14 oder SEQ ID No.16 gegen L-Threonin; siehe Omori et al. (Journal of Bacteriology 175(3), 785-794 (1993)),
umfasst.

Bevorzugt wird eines der thrA^{FBR}-Allele thrA E253K (Austausch von L-Glutaminsäure an Position 253 des kodierten Enzyms Aspartatkinase/Homoserin-Dehydrogenase gegen L-Lysin)oder S345F (Austausch von L-Serin an Position 345 des kodierten Enzyms Aspartatkinase/Homoserin-Dehydrogenase gegen L-Phenylalanin) gemäß SEQ ID No.14.

Die hier beschriebenen thrA^{FBR}-Allele, die für ein Aspartatkinase/Homoserin-Dehydrogenase Enzym kodieren, können mit den oben beschriebenen Maßnahmen überexprimiert werden.

Gegebenenfalls kann es von Vorteil sein, nur eine gemäßigte Erhöhung der intrazellulären Aktivität oder Konzentration eines oder mehrerer Enzyme oder Proteine in einem Mikroorganismus durchzuführen, da eine zu starke Erhöhung zum Beispiel zu einer fehlerhaften Zellteilung oder veränderter Zellmorphologie bis hin zur Toxizität führen kann (Guthrie und Wickner, Journal of Bacteriology 172(10):5555-5562 (1990); Genevaux P. et al.; EMBO Reports 5(2) : 195-200 (2004)).

Der Begriff "gemäßigte Erhöhung" beschreibt die Erhöhung der intrazellulären Aktivität oder Konzentration des entsprechenden Proteins um höchstens das 10-fache, das 8-fache, das 6-fache, das 4-fache, das 3-fache, das 2-fache oder das 1,5-fache bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm. Als nicht rekombinanter Mikroorganismus oder Elternstamm (parent strain) wird der Mikroorganismus verstanden, an dem die erfindungsgemäße Verstärkung oder Überexpression durchgeführt wird.

Weiterhin kann es für die Produktion von L-Aminosäuren, insbesondere L-Threonin, L-Tryptophan, L-Lysin, L-Valin, L-Isoleucin und L-Homoserin vorteilhaft sein, zusätzlich zur Verstärkung des can-Gens und/oder des cynT-Gens, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

So können beispielsweise für die Produktion von L-Threonin gleichzeitig eines oder mehrere der Gene, ausgewählt aus der Gruppe
das für die Threonin-Dehydrogenase kodierende tdh-Gen (Journal of Bacteriology 169: 4716-4721 (1987)),
das für die Malat-Dehydrogenase (E.C. 1.1.1.37) kodierende mdh-Gen (Archives in Microbiology 149: 36-42 (1987)),
das für das Enzym Phosphoenolpyruvat-Carboxykinase kodierende pckA-Gen (WO 02/29080),
das für die Pyruvat-Oxidase kodierende poxB-Gen (WO 02/36797),
das für den DgsA-Regulator des Phosphotransferase-Systems kodierende dgsA-Gen (WO 02/081721), das auch unter der Bezeichnung mlc-Gen bekannt ist,
das für den Fructose-Repressor kodierende fruR-Gen (WO 02/081698), das auch unter der Bezeichnung cra-Gen bekannt ist,
das für den Sigma³⁸- Faktor kodierende rpoS-Gen (WO 01/05939), das auch unter der Bezeichnung katF-Gen bekannt ist,
das für die Aspartat Ammonium-Lyase kodierende aspA-Gen (WO 03/008603),
abgeschwächt, insbesondere ausgeschaltet oder die Expression verringert werden. Diese Massnahmen werden gegebenenfalls zusätzlich zu bzw. in geeigneter Kombination mit den angegebenen Massnahmen der Verstärkung von Genen zur Erhöhung der Threonin-Produktion durchgeführt.

Weiterhin kann es für die Produktion von L-Tryptophan vorteilhaft sein, zusätzlich zur Verstärkung des uspA-Gens, eines oder mehrere der Gene ausgewählt aus der Gruppe
das für die Tryptophanase kodierende tnaA-Gen (US4, 371, 614),
das für den Repressor des trp-Operons kodierende trpR-Gen (US4,371,614)
das für die Chorismat-Mutase T und Prephenat-Dehydrogenase kodierende tyrA-Gen (US4,371,614),
das für die Chorismat-Mutase P und Prephenat-Dehydrogenase kodierende pheA-Gen (US4,371,614),
das für das Tryptophan spezifische Transportprotein kodierende mtr-Gen (US5,756,345),
das für die Tryptophan-Permease kodierende tnaB-Gen (USS, 756, 345),
das für den Transporter für aromatische Aminosäuren kodierende aroP-Gen (US5,756,345),
das für die L-Serin Deaminase kodierende sdaA-Gen (EP0149539),
das für die Glukose-6-Phosphat-Isomerase kodierende pgi-Gen (WO87/01130),
das für die Tyrosin-Aminotransferase kodierende tyrB-Gen (WO87/01130),
abzuschwächen, insbesondere auszuschalten oder die Expression zu verringern. Diese Massnahmen werden gegebenenfalls zusätzlich zu bzw. in geeigneter Kombination mit den angegebenen Massnahmen der Verstärkung von Genen zur Erhöhung der Tryptophan-Produktion durchgeführt.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Gegebenenfalls können die Maßnahmen zur Verstärkung und zur Abschwächung beliebig kombiniert werden.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben erhöht.

Erfindungsgemäß werden die hergestellten Mikroorganismen im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert. Zusammenfassungen über derartige Verfahren sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Bei einem batch-Verfahren werden, mit wenigen Ausnahmen, wie beispielsweise Sauerstoff und pH-Korrekturmittel, sämtliche Einsatzstoffe in Form eines Ansatzes vorgelegt und der Mikrorganismus in dem erhaltenen Medium kultiviert.

Bei einem fed batch-Verfahren wird der Mikrorganismus zunächst mittels eines batch-Verfahrens kultiviert (batch-Phase). Im Anschluss daran wird der Kultur ein für die Herstellung des Produktes wesentlicher Einsatzstoff, ggf. auch mehrere Einsatzstoffe, kontinuierlich oder diskontinuierlich hinzugefügt (Zulaufphase, feed-Phase). Im Falle der Herstellung von L-Aminosäuren handelt es sich hierbei um eine Kohlenstoffquelle.

Bei einem repeated fed batch-Verfahren handelt es sich um ein fed batch-Verfahren, bei dem nach Abschluss der Fermentation ein Teil der erhaltenen Fermentationsbrühe als Inokulum zum Start eines erneuten repeated fed batch-Verfahrens dient. Dieser Zyklus kann ggf. mehrfach wiederholt werden. Repeated fed batch-Verfahren sind beispielsweise in der WO 02/18543 und WO 05/014843 beschrieben.

Bei einem kontinuierlichen Verfahren werden im Anschluß an ein batch- oder fed batch-Verfahren ein oder mehrere ggf. sämtliche Einsatzstoffe zu der Kultur kontinuierlich hinzugefügt und gleichzeitig Fermentationsbrühe entnommen. Kontinuierliche Verfahren sind beispielsweise in den Patentschriften US 5,763,230, WO 05/014840, WO 05/014841 und WO 05/014842 beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Im Allgemeinen enthält ein Kulturmedium unter anderem eine oder mehrere Kohlenstoffquelle(n), Stickstoffquelle(n) und Phosphorquelle(n).

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einzigen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung bzw. Akkumulation der L-Aminosäure in der Fermentations- bzw. Kulturbrühe. Die Kultur wird solange fortgesetzt, bis sich ein Maximum an der gewünschten L-Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Unter einer Fermentationsbrühe bzw. Kulturbrühe versteht man ein Fermentationsmedium, in dem ein Mikroorganismus für eine gewisse Zeit und bei einer gewissen Temperatur kultiviert wurde.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete L-Aminosäure, c) die im Laufe der Fermentation gebildeten organischen Nebenprodukte und d) die durch die Fermentation nicht verbrauchten Bestandteile des/ der eingesetzten Fermentationsmediums/ Fermentationsmedien bzw. der Einsatzstoffe wie beispielsweise Vitamine wie Thiamin oder Salze wie Magnesiumsulfat.

Die hergestellte Kultur- bzw. Fermentationsbrühe kann anschließend gesammelt und die gewünschte L-Aminosäure bzw. das L-Aminosäure-haltige Produkt gewonnen oder isoliert werden.

In einer Verfahrensvariante wird die Fermentationsbrühe gegebenenfalls konzentriert und anschließend die L-Aminosäure gereinigt bzw. in reiner oder nahezu reiner Form isoliert. Typische Methoden zur Reinigung von L-Aminosäuren sind die Ionenaustauschchromatographie, die Kristallisation, Extraktionsverfahren und die Behandlung mit Aktivkohle. Hierdurch erhält man weitgehend reine L-Aminosäuren mit einem Gehalt von 90 Gew.-%, ≥ 95 Gew.-%, ≥ 96 Gen.-%, ≥ 97 Gen.-% ≥ 98 Gew.-% oder ≥ 99 Gew.-%.

Es ist ebenfalls möglich in einer anderen Verfahrensvariante aus der hergestellten Fermentationsbrühe ein Produkt herzustellen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder Granulier-Verfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von L-Aminosäuren zur Bestimmung der Konzentration zu einem oder mehreren Zeitpunkt(en) im Verlauf der Fermentation kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

## Patentansprüche

1. Rekombinante L-Threonin oder L-Tryptophan produzierende Mikroorganismen der Familie Enterobacteriaceae, die ein verstärktes oder überexprimiertes can-Gen und/oder cynT-Gen enthalten, welche für Carboanhydrasen kodieren, wobei die L-Threonin produzierenden Mikroorganismen zusätzlich eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pyridin-Transhydrogenase kodierenden Gene pntA und pntB,
f) das Gen rhtC, welches für das Threoninresistenz vermittelnde Protein kodiert,
g) das für das Threonin-Export-Carrier-Protein kodierende thrE-Gen aus Corynebacterium glutamicum,
h) das für die Glutamat-Dehydrogenase kodierende gdhAGen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die β-Untereinheit der Succinyl-CoA Synthetase kodierende sucC-Gen,
u) das für die α-Untereinheit der Succinyl-CoA Synthetase kodierende sucD-Gen,
v) das Genprodukt des offenen Leserahmens (ORF) yjcG von Escherichia coli,
w) das Genprodukt des offenen Leserahmens (ORF) yibD von Escherichia coli,
x) das Genprodukt des offenen Leserahmens (ORF) yaaU von Escherichia coli,
y) das für das universelle Stressprotein UspC kodierende uspC-Gen,
z) das uspD-Gen, welches für das Resistenz gegen UV-Strahlung vermittelnde universelle Stressprotein UspD kodiert und
aa) das uspE-Gen, welches für das Resistenz gegen UV-Strahlung vermittelnde universelle Stressprotein UspE kodiert,
verstärkt oder überexprimiert enthalten, und die L-Tryptophan produzierenden Mikroorganismen zusätzlich gleichzeitig eines oder mehrere der Gene, ausgewählt aus der Gruppe:
a) mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons,
b) das für die 3-Phosphoglycerat-Dehydrogenase kodierende serA-Gen,
c) das für die Phosphoserinphosphatase kodierende serB-Gen,
d) das für die Phosphoserinaminotransferase kodierende serC-Gen,
e) das für die L-Tyrosin sensitive DHAP-Synthase kodierende aroF-Gen,
f) das für die L-Phenylalanin sensitive DHAP-Synthase kodierende aroG-Gen,
g) das für die L-Tryptophan sensitive DHAP-Synthase kodierende aroH-Gen,
h) das für die Phosphoenolpyruvat-Synthase kodierende pps-Gen,
i) das für die Phosphoenolpyruvat-Carboxykinase kodierende pckA-Gen,
j) das für die Transketolase A kodierende tktA-Gen,
k) das für die Transketolase B kodierende tktB-Gen,
1) das Genprodukt des offenen Leserahmens (ORF) yddG von Escherichia coli,
m) das für das universelle Stressprotein UspC kodierende uspC-Gen,
n) das für das Resistenz gegen UV-Strahlung vermittelnde universelle Stressprotein UspD kodierende uspD-Gen und
o) das für das Resistenz gegen UV-Strahlung vermittelnde universelle Stressprotein UspE kodierende uspE-Gen,
verstärkt oder überexprimiert enthalten.

2. Mikroorganismen gemäss Anspruch 1, herstellbar durch einzelne oder gemeinsame Verstärkung der als can-Gen und als cynT-Gen bezeichneten Polynukleotide, wobei
a) das can-Gen für ein Polypeptid kodiert, dessen Aminosäuresequenz zwischen den Positionen 57 und 98 oder vergleichbaren Positionen die Sequenz ProGlyXaaLeuPheValHisArgAsnValAlaAsnLeuValIleHisT hrAspLeuAsnCysLeuSerValXaaGlnTyrAlaValXaaValLeuGl uValGluHisIleIleIleCysGlyHis (SEQ ID No. 17) enthält, wobei an Position 1 der Aminosäuresequenz Methionin steht, kodiert durch das Startcodon AUG oder GUG, und Xaa eine beliebige Aminosäure sein kann,
und das im Wesentlichen eine Länge von 220 Aminosäuren umfasst
und
b) das cynT-Gen für ein Polypeptid kodiert, dessen Aminosäuresequenz zwischen den Positionen 50 und 71 oder vergleichbaren Positionen die Sequenz ThrGlnXaaGluProGlyXaaLeuPheValIleArgAsnAlaGlyAsnI leValProSerXaaGly (SEQ ID No. 18) enthält, wobei an Position 1 der Aminosäuresequenz Methionin steht, kodiert durch das Startcodon AUG oder GUG, und Xaa eine beliebige Aminosäure sein kann,
und das im Wesentlichen eine Länge von 219 Aminosäuren umfasst,
wobei die Polypeptide die Aktivität einer Carboanhydrase besitzen.

3. Mikroorganismen gemäss Anspruch 1, in denen als can-Gen und cynT-Gen bezeichnete Polynukleotide einzeln oder gemeinsam verstärkt werden, wobei can- und cynT-Gen ausgewählt sind aus der Gruppe, bestehend aus:
a) can-Gen, das für ein Polypeptid kodiert, dessen Aminosäuresequenz zu mindestens 80% identisch ist mit einer Aminosäuresequenz, ausgewählt aus der Gruppe SEQ ID No. 2, SEQ ID No. 4 und SEQ ID No. 6 und
b) das cynT-Gen für ein Polypeptid kodiert, dessen Aminosäuresequenz zu mindestens 80% identisch ist mit einer Aminosäuresequenz, ausgewählt aus der Gruppe SEQ ID No. 8, SEQ ID No. 10 und SEQ ID No. 12,
c) das can-Gen, das für ein Polypeptid kodiert, dessen Aminosäuresequenz zu mindestens 95% identisch ist mit einer Aminosäuresequenz, ausgewählt aus der Gruppe SEQ ID No. 2, SEQ ID No. 4 und SEQ ID No. 6, und
d) das cynT-Gen, das für ein Polypeptid kodiert, dessen Aminosäuresequenz zu mindestens 95% identisch ist mit einer Aminosäuresequenz, ausgewählt aus der Gruppe SEQ ID No. 8, SEQ ID No. 10 und SEQ ID No. 12, wobei die Polypeptide die Aktivität einer Carbonhydrase besitzen,
e) das cynT- oder can-Gen, die für Polypeptide kodieren, deren Aminosäuresequenzen zu 100% identisch sind mit denen der Sequenzen SEQ ID No. 2 oder SEQ ID No. 8.

4. Mikroorganismen gemäss Anspruch 3, **dadurch gekennzeichnet, dass** sie als can-Gen und als cynT-Gen bezeichnete isolierte Polynukleotide, einzeln oder gemeinsam verstärkt, enthalten, ausgewählt aus der Gruppe:
a) can-Gen mit einer Nukleotidsequenz, ausgewählt aus SEQ ID No. 1, SEQ ID No. 3, SEQ ID No. 5 und cynT-Gen mit einer Nukleotidsequenz, ausgewählt aus SEQ ID No. 7, SEQ ID No. 9, SEQ ID No. 11 und den dazu komplementären Nukleotidsequenzen;
b) can-Gen mit einer Nukleotidsequenz, ausgewählt aus den Sequenzen, die der SEQ ID No. 1, SEQ ID No. 3 oder SEQ ID No. 5 im Rahmen der Degeneration des genetischen Codes entsprechen, und cynT-Gen mit einer Nukleotidsequenz, ausgewählt aus den Sequenzen, die der SEQ ID No. 7, SEQ ID No. 9 oder SEQ ID No. 11 im Rahmen der Degeneration des genetischen Codes entsprechen;
c) can-Gen mit einer Sequenz, ausgewählt aus den Sequenzen, die mit den zu den Sequenzen SEQ ID No. 1, SEQ ID No. 3 oder SEQ ID No. 5 komplementären Sequenzen unter stringenten Bedingungen hybridisieren, und cynT-Gen mit einer Nukleotidsequenz, ausgewählt aus den Sequenzen, die mit den zu den Sequenzen SEQ ID No. 7, SEQ ID No. 9, SEQ ID No. 11 komplementären Sequenzen unter stringenten Bedingungen hybridisieren,
wobei die stringenten Bedingungen durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) can-Gen mit einer Nukleotidsequenz, für das can-Gen ausgewählt aus den Sequenzen SEQ ID No. 1, SEQ ID No. 3 und SEQ ID No. 5, das cynT-Gen mit einer Nukleotidsequenz, ausgewählt aus den Sequenzen SEQ ID No. 7, SEQ ID No. 9, und SEQ ID No. 11, die funktionsneutrale Sinnmutanten enthalten,
wobei die Polynukleotide für Carboanhydrasen kodieren.

5. Mikroorganismus gemäss den Ansprüchen 1 bis 4, **dadurch gekennzeichnet, dass** sie durch Transformation, Transduktion, Konjugation oder eine Kombination dieser Methoden hergestellt werden, mit einem Vektor, der das genannte can-Gen und/oder cynT-Gen, ein Allel dieser Gene oder Teile davon und/oder einen Promotor enthält.

6. Mikroorganismen gemäss den Ansprüchen 1 bis 5, in denen die Kopienzahl des genannten can-Gen und/oder cynT-Gens oder der Allele um mindestens 1 erhöht vorliegt.

7. Mikroorganismen gemäss Anspruch 6, **dadurch gekennzeichnet, dass** die Erhöhung der Kopienzahl des genannten can-Gen und/oder cynT-Gens um mindestens 1 durch Integration des Gens oder der Allele in das Chromosom der Mikroorganismen bewirkt wird.

8. Mikroorganismen gemäss Anspruch 7, **dadurch gekennzeichnet, dass** die Erhöhung der Kopienzahl des genannten can-Gen und/oder cynT-Gens um mindestens 1 durch einen extrachromosomal replizierenden Vektor erzielt wird.

9. Mikroorganismen gemäss den Ansprüchen 1 bis 8, **dadurch gekennzeichnet, dass** man zur Erzielung der Verstärkung
a) die Promotor- und Regulationsregion oder die Ribosomenbindungsstelle stromaufwärts des genannten can-Gen und/oder cynT-Gens mutiert, oder
b) Expressionskassetten oder Promotoren stromaufwärts des can-Gen und/oder cynT-Gens einbaut.

10. Mikroorganismen gemäss den Ansprüchen 1 bis 9, **dadurch gekennzeichnet, dass** die Expression des genannten can-Gen und/oder cynT-Gens unter der Kontrolle eines die Expression des Gens verstärkenden Promotors steht.

11. Mikroorganismen gemäss den Ansprüchen 1 bis 10,
**dadurch gekennzeichnet, dass** durch die Verstärkung des genannten can-Gens und/oder cynT-Gens die Konzentrationen oder Aktivitäten der kodierten Genprodukte (Proteine) um mindestens 10% erhöht sind, bezogen auf die Aktivität oder Konzentration des Genproduktes im für das can-Gen und/oder cynT-Gen nicht rekombinanten Mikroorganismus oder Elternstamm.

12. Mikroorganismen gemäss den Ansprüchen 1 bis 11,
**dadurch gekennzeichnet, dass** die Mikroorganismen aus den Gattungen Escherichia, Erwinia, Providencia und Serratia ausgewählt sind.

13. Verfahren zur Herstellung von L-Aminosäuren durch Fermentation von rekombinanten Mikroorganismen der Familie Enterobacteriaceae, **dadurch gekennzeichnet, dass** man
a) die die gewünschte L-Aminosäure produzierenden Mikroorganismen gemäß den Ansprüchen 1 bis 12 in einem Medium kultiviert unter Bedingungen, bei denen die gewünschte L-Aminosäure im Medium oder in den Zellen angereichert wird, und
b) die gewünschte L-Aminosäure isoliert, wobei Bestandteile der Fermentationsbrühe und/oder die Biomasse in ihrer Gesamtheit oder Anteilen (≥ 0 bis 100%) im isolierten Produkt verbleiben oder vollständig entfernt werden.

14. Verfahren gemäss Anspruch 13, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das für die Phosphoenolpyruvat-Carboxykinase kodierende pckA-Gen,
d) das für die Pyruvat-Oxidase kodierende poxB-Gen,
e) das für den DgsA-Regulator des Phosphotransferase-Systems kodierende dgsA-Gen,
f) das für den Fructose-Repressor kodierende fruR-Gen,
g) das für den Sigma³⁸-Faktor kodierende rpoS-Gen, und
h) das für die Aspartat Ammonium-Lyase kodierende
aspA-Gen,
abschwächt, insbesondere ausschaltet oder die Expression verringert.

15. Verfahren gemäss Anspruch 13, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
a) das für die Tryptophanase kodierende tnaA-Gen,
b) das für den Repressor des trp-Operons kodierende trpR-Gen,
c) das für die Chorismat-Mutase T und Prephenat-Dehydrogenase kodierende tyrA-Gen,
d) das für die Chorismat-Mutase P und Prephenat-Dehydrogenase kodierende pheA-Gen,
e) das für das Tryptophan spezifische Transportprotein kodierende mtr-Gen,
f) das für die Tryptophan-Permease kodierende tnaB-Gen,
g) das für den Transporter für aromatische Aminosäuren kodierende aroP-Gen,
h) das für die L-Serin Deaminase kodierende sdaA-Gen,
i) das für die Glukose-6-Phosphat-Isomerase kodierende pgi-Gen, und
j) das für die Tyrosin-Aminotransferase kodierende
tyrB-Gen,
abschwächt, insbesondere ausschaltet oder die Expression verringert.

16. Verfahren gemäss Anspruch 13, **dadurch gekennzeichnet, dass** man L-Aminosäuren ausgewählt aus L-Threonin und L-Tryptophan herstellt.

17. Verfahren gemäss Anspruch 13 - 16, **dadurch gekennzeichnet, dass** die Konzentration der L-Aminosäure zu einem oder mehreren Zeitpunkt(en) im Verlauf der Fermentation bestimmt wird.

## Claims

1. Recombinant L-threonine- or L-tryptophan-producing microorganisms of the family Enterobacteriaceae, which contain an enhanced or overexpressed can gene and/or cynT gene which code for carbonic anhydrases, in which L-threonine-producing microorganisms additionally comprise one or more of the genes selected from the group consisting of:
a) at least one gene of the thrABC operon coding for aspartate kinase, homoserine dehydrogenase, homoserine kinase and threonine synthase,
b) the pyc gene of Corynebacterium glutamicum coding for pyruvate carboxylase,
c) the pps gene coding for phosphoenol pyruvate synthase,
d) the ppc gene coding for phosphoenol pyruvate carboxylase,
e) the genes pntA and pntB coding for the subunits of pyridine transhydrogenase,
f) the gene rhtC, which codes for the threonine resistance-mediating protein,
g) the thrE gene from Corynebacterium glutamicum coding for the threonine export carrier protein,
h) the gdhA gene coding for glutamate dehydrogenase,
i) the ptsH gene coding for the phosphohistidine protein hexose phosphotransferase,
j) the ptsI gene coding for the enzyme I of the phosphotransferase system,
k) the crr gene coding for the glucose-specific IIA component,
l) the ptsG gene coding for the glucose-specific IIBC component,
m) the cysK gene coding for cysteine synthase A,
n) the cysB gene coding for the regulator of the cys regulon,
o) the cysJ gene coding for the flavoprotein of NADPH sulphite reductase,
p) the cysI gene coding for the haemoprotein of NADPH sulphite reductase,
q) the cysH gene coding for adenylyl sulphate reductase,
r) the sucA gene coding for the decarboxylase subunit of 2-ketoglutarate dehydrogenase,
s) the sucB gene coding for the dihydrolipoyltranssuccinase E2 subunit of 2-ketoglutarate dehydrogenase,
t) the sucC gene coding for the β-subunit of succinyl-CoA synthetase,
u) the sucD gene coding for the α-subunit of succinyl-CoA synthetase,
v) the gene product of the open reading frame (ORF) yjcG of Escherichia coli,
w) the gene product of the open reading frame (ORF) yibD of Escherichia coli,
x) the gene product of the open reading frame (ORF) yaaU of Escherichia coli,
y) the uspC gene coding for the universal stress protein UspC,
z) the uspD gene, which codes for the resistance against UV radiation-mediating universal stress protein UspD and
aa) the uspE gene, which codes for the resistance against UV radiation-mediating universal stress protein UspE,
in enhanced or overexpressed form, and the L-tryptophan-producing microorganisms additionally at the same time comprise one or more of the genes selected from the group consisting of:
a) at least one gene of the tryptophan operon coding for anthranilate synthase, anthranilate phosphoribosyl transferase, phosphoribosyl anthranilate isomerase, indole-3-glycerol phosphate synthase and tryptophan synthase,
b) the serA gene coding for 3-phosphoglycerate dehydrogenase,
c) the serB gene coding for phosphoserine phosphatase,
d) the serC gene coding for phosphoserine aminotransferase,
e) the aroF gene coding for L-tyrosine-sensitive DHAP synthase,
f) the aroG gene coding for L-phenylalanine-sensitive DHAP synthase,
g) the aroH gene coding for L-tryptophan-sensitive DHAP synthase,
h) the pps gene coding for phosphoenol pyruvate synthase,
i) the pckA gene coding for phosphoenol pyruvate carboxykinase,
j) the tktA gene coding for transketolase A,
k) the tktB gene coding for transketolase B,
1) the gene product of the open reading frame (ORF) yddG of Escherichia coli,
m) the uspC gene coding for the universal stress protein UspC,
n) the uspD gene coding for the resistance against UV radiation-mediating universal stress protein UspD and
o) the uspE gene coding for the resistance against UV radiation-mediating universal stress protein UspE,
in enhanced or overexpressed form.

2. Microorganisms according to Claim 1, producible by enhancing individually or jointly the polynucleotides referred to as can gene and cynT gene, where
a) the can gene codes for a polypeptide, the amino acid sequence of which comprises, between positions 57 and 98 or comparable positions, the sequence
ProGlyXaaLeuPheValHisArgAsnValAlaAsnLeuVal-IleHisThrAspLeuAsnCysLeuSerValXaaGlnTyrAlaVal XaaValLeuGluValGluHisIleIleIleCysGlyHis (SEQ ID No. 17), where methionine is in position 1 of the amino acid sequence, encoded by the start codon AUG or GUG, and Xaa may be any amino acid,
and which essentially is 220 amino acids in length,
and
b) the cynT gene codes for a polypeptide, the amino acid sequence of which comprises, between positions 50 and 71 or comparable positions, the sequence
ThrGlnXaaGluProGlyXaaLeuPheValIleArgAsnAla-GlyAsnIleValProSerXaaGly (SEQ ID No. 18), where methionine is in position 1 of the amino acid sequence, encoded by the start codon AUG or GUG, and Xaa may be any amino acid,
and which essentially is 219 amino acids in length,
where the polypeptides have the activity of a carbonic anhydrase.

3. Microorganisms according to Claim 1, in which polynucleotides referred to as can gene and cynT gene are enhanced individually or jointly, where can and cynT genes are selected from the group consisting of:
a) can gene which codes for a polypeptide, the amino acid sequence of which is at least 80% identical to an amino acid sequence selected from the group of SEQ ID No. 2, SEQ ID No. 4 and SEQ ID No. 6, and
b) the cynT gene coding for a polypeptide, the amino acid sequence of which is at least 80% identical to an amino acid sequence selected from the group of SEQ ID No. 8, SEQ ID No. 10 and SEQ ID No. 12,
c) the can gene which codes for a polypeptide, the amino acid sequence of which is at least 95% identical to an amino acid sequence selected from the group of SEQ ID No. 2, SEQ ID No. 4 and SEQ ID No. 6, and
d) the cynT gene which codes for a polypeptide, the amino acid sequence of which is at least 95% identical to an amino acid sequence selected from the group of SEQ ID No. 8, SEQ ID No. 10 and SEQ ID No. 12, where the polypeptides have the activity of a carbonic anhydrase,
e) the cynT gene or can gene which code for polypeptides, the amino acid sequences of which are 100% identical to the sequences SEQ ID No. 2 or SEQ ID No. 8.

4. Microorganisms according to Claim 3, **characterized in that** they contain isolated polynucleotides referred to as can gene and cynT gene, individually or jointly enhanced, selected from the group consisting of:
a) can gene having a nucleotide sequence selected from SEQ ID No. 1, SEQ ID No. 3, SEQ ID No. 5, and cynT gene having a nucleotide sequence selected from SEQ ID No. 7, SEQ ID No. 9, SEQ ID No. 11, and the nucleotide sequences complementary thereto;
b) can gene having a nucleotide sequence selected from the sequences that correspond to SEQ ID No. 1, SEQ ID No. 3 or SEQ ID No. 5 within the degeneracy of the genetic code, and cynT gene having a nucleotide sequence selected from the sequences that correspond to SEQ ID No. 7, SEQ ID No. 9 or SEQ ID No. 11 within the degeneracy of the genetic code;
c) can gene having a sequence selected from the sequences that hybridize under stringent conditions with the sequences complementary to the sequences SEQ ID No. 1, SEQ ID No. 3 or SEQ ID No. 5, and cynT gene having a nucleotide sequence selected from the sequences that hybridize under stringent conditions with the sequences complementary to the sequences SEQ ID No. 7, SEQ ID No. 9, SEQ ID No. 11,
where the stringent conditions are achieved by a washing step in which the temperature range is from 64°C to 68°C, and the range of salt concentrations of the buffer is from 2×SSC to 0.1×SSC;
d) can gene having a nucleotide sequence, for the can gene selected from the sequences SEQ ID No. 1, SEQ ID No. 3 and SEQ ID No. 5, the cynT gene having a nucleotide sequence selected from the sequences SEQ ID No. 7, SEQ ID No. 9, and SEQ ID No. 11, which contain functionally neutral sense mutations,
where the polynucleotides code for carbonic anhydrases.

5. Microorganisms according to one of Claims 1 to 4, **characterized in that** they are produced by transformation, transduction, conjugation or a combination of these methods with a vector that contains the abovementioned can gene and/or cynT gene, an allele of these genes or parts thereof and/or a promoter.

6. Microorganisms according to Claims 1 to 5, in which the copy number of the abovementioned can gene and/or cynT gene or of the alleles has been increased by at least 1.

7. Microorganisms according to Claim 6, **characterized in that** the increase in the copy number of the abovementioned can gene and/or cynT gene by at least 1 is brought about by integrating the gene or the alleles into the chromosome of the microorganisms.

8. Microorganisms according to Claim 7, **characterized in that** the increase in the copy number of the abovementioned can gene and/or cynT gene by at least 1 is achieved by means of a vector replicating extrachromosomally.

9. Microorganisms according to Claims 1 to 8, **characterized in that** enhancement is achieved by
a) mutating the promoter and regulatory regions or the ribosome binding site upstream of the abovementioned can gene and/or cynT gene, or
b) incorporating expression cassettes or promoters upstream of the can gene and/or cynT gene.

10. Microorganisms according to Claims 1 to 9, **characterized in that** expression of the abovementioned can gene and/or cynT gene is under the control of a promoter enhancing expression of said gene.

11. Microorganisms according to Claims 1 to 10, **characterized in that** enhancement of the abovementioned can gene and/or cynT gene increases the concentrations or activities of the encoded gene products (proteins) by at least 10%, based on the activity or concentration of the gene product in the microorganism or parent strain that is non-recombinant for the can gene and/or cynT gene.

12. Microorganisms according to Claims 1 to 11, **characterized in that** the microorganisms are selected from the genera Escherichia, Erwinia, Providencia and Serratia.

13. Process for the production of L-amino acids by fermentation of recombinant microorganisms of the family Enterobacteriaceae, **characterized in that**
a) the microorganisms producing the desired L-amino acid according to Claims 1 to 12 are cultured in a medium under conditions in which the desired L-amino acid is enriched in the medium or in the cells, and
b) the desired L-amino acid is isolated, where constituents of the fermentation broth and/or the biomass remain in the isolated product in their entirety or in part (≥ 0 to 100%) or are completely removed.

14. Process according to Claim 13, **characterized in that** for the production of L-threonine microorganisms are fermented, in which additionally at the same time one or more of the genes selected from the group consisting of:
a) the tdh gene coding for threonine dehydrogenase,
b) the mdh gene coding for malate dehydrogenase,
c) the pckA gene coding for phosphoenol pyruvate carboxykinase,
d) the poxB gene coding for pyruvate oxidase,
e) the dgsA gene coding for the DgsA regulator of the phosphotransferase system,
f) the fruR gene coding for the fructose repressor,
g) the rpoS gene coding for the Sigma³⁸ factor, and
h) the aspA gene coding for aspartate ammonium
lyase,
are attenuated, in particular switched off, or the expression is decreased.

15. Process according to Claim 13, **characterized in that** for the production of L-tryptophan microorganisms of the family Enterobacteriaceae are fermented, in which additionally at the same time one or more of the genes selected from the group consisting of:
a) the tnaA gene coding for tryptophanase,
b) the trpR gene coding for the repressor of the trp operon,
c) the tyrA gene coding for chorismate mutase T and prephenate dehydrogenase,
d) the pheA gene coding for chorismate mutase P and prephenate dehydrogenase,
e) the mtr gene coding for the tryptophan-specific transport protein,
f) the tnaB gene coding for tryptophan permease,
g) the aroP gene coding for the transporter for aromatic amino acids,
h) the sdaA gene coding for L-serine deaminase,
i) the pgi gene coding for glucose-6-phosphate isomerase, and
j) the tyrB gene coding for tyrosine
aminotransferase,
are attenuated, in particular switched off, or the expression is decreased.

16. Process according to Claim 13, **characterized in that** L-amino acids selected from L-threonine and L-tryptophan are produced.

17. Process according to Claims 13-16, **characterized in that** the concentration of the L-amino acid is determined at one or more point(s) in time in the course of the fermentation.

## Revendications

1. Micro-organismes recombinants de la famille des Enterobacteriaceae, produisant de la L-thréonine ou du L-tryptophane, qui contiennent un gène can et/ou un gène cynT amplifié ou surexprimé, lesquels codent pour des carboanhydrases, les micro-organismes produisant la L-thréonine contenant en outre, amplifiés ou surexprimés, un ou plusieurs gènes choisis dans le groupe :
a) au moins un gène de l'opéron thrABC, codant pour l'aspartatekinase, l'homosérine-déshydrogénase, l'homosérinekinase et la thréoninesynthase,
b) la gène pyc de Corynebacterium glutamicum, codant pour la pyruvatecarboxylase,
c) le gène pps codant pour la phosphoénolpyruvate-synthase,
d) le gène ppc codant pour la phosphoénolpyruvate-carboxylase,
e) les gènes pntA et pntB codant pour les sous-unités de la pyrdine-transhydrogénase,
f) le gène rhtC, qui code pour la protéine conférant une résistance à la thréonine,
g) le gène thrE de Corynebacterium glutamicum, codant pour la protéine transporteuse d'exportation de la thréonine,
h) le gène gdhA codant pour la glutamate-déshydrogénase,
i) le gène ptsH codant pour la phosphohistidine-protéine-hexose-phosphotransférase,
j) le gène ptsI codant pour l'enzyme I du système phosphotransférase,
k) le gène crr codant pour le composant IIA spécifique du glucose,
l) le gène ptsG codant pour le composant IIBC spécifique du glucose,
m) le gène cysK codant pour la cystéinesynthase A,
n) le gène cysB codant pour le régulateur du régulon cys,
o) le gène cysJ codant pour la flavoprotéine de la NADPH-sulfite-réductase,
p) le gène cysI codant pour l'hémoprotéine de la NADPH-sulfite-réductase,
q) le gène cysH codant pour l'adénylylsulfate-réductase,
r) le gène sucA codant pour la sous-unité décarboxylase de la 2-cétoglutarate déshydrogénase,
s) le gène sucB codant pour la sous-unité dihydrolipoyltranssuccinase E2 de la 2-cétoglutarate déshydrogénase,
t) le gène sucC codant pour la sous-unité β de la succinyl-CoA synthétase,
u) le gène sucD codant pour la sous-unité α de la succinyl-CoA synthétase,
v) le produit génique du cadre de lecture ouvert (ORF) yjcG d'Escherichia coli,
w) le produit génique du cadre ouvert de lecture (ORF) yibD d'Escherichia coli,
x) le produit génique du cadre ouvert de lecture (ORF) yaaU d'Escherichia coli,
y) le gène uspC codant pour la protéine de stress universelle UspC,
z) le gène uspD, qui code pour la protéine de stress universelle UspD conférant une résistance au rayonnement UV, et
aa) le gène uspE, qui code pour la protéine de stress universelle UspE conférant une résistance au rayonnement UV,
et les micro-organismes produisant le L-tryptophane contenant, amplifiés ou surexprimés, en plus et simultanément un ou plusieurs des gènes choisis dans le groupe comprenant :
a) au moins un gène de l'opéron tryptophane codant pour l'anthranilate-synthase, l'anthralinate-phosphoribosyl-transférase, la phosphoribosylanthranilate-isomérase, l'indole-3-glycérinephosphate-synthase, et la tryptophane-synthase,
b) le gène serA codant pour la 3-phosphoglycérate-déhydrogénase,
c) le gène serB codant pour la phosphosérine-phosphatase,
d) le gène serC codant pour la phosphosérine-aminotransférase,
e) le gène aroF codant pour la DHAP-synthase sensible à la L-tyrosine,
f) le gène aroG codant pour la DHAP-synthase sensible à la L-phénylalanine,
g) le gène aroH codant pour la DHAP-synthase sensible au L-tryptophane,
h) le gène pps codant pour la phosphoénolpyruvate-synthase,
i) le gène pckA codant pour la phosphoénolpyruvate-carboxykinase,
j) le gène tktA codant pour la transkétolase A,
k) le gène tktB codant pour la transkétolase B,
1) le produit génique du cadre ouvert de lecture (ORF) yddG d'Escherichia coli,
m) le gène uspC codant pour la protéine de stress universelle UspC,
n) le gène uspD codant pour la protéine de stress universelle UspD conférant une résistance au rayonnement UV, et
o) le gène uspE codant pour la protéine de stress universelle UspE conférant une résistance au rayonnement UV.

2. Micro-organismes selon la revendication 1, pouvant être fabriqués par une amplification, individuelle ou commune, des polynucléotides désignés sous le nom de gène can et de gène cynT, dans lesquels :
a) le gène can code pour un polypeptide dont la séquence d'acides aminés contient, entre les positions 57 et 98 ou des positions comparables, la séquence ProGlyXaaLeuPheValHisArgAsnValAlaAsnLeuValIleHisThrAspL euAsnCysLeuSerValXaaGlnTyrAlaValXaaValLeuGluValGluHisIl eIleIleCysGlyHis (SEQ ID N° 17), une méthionine se trouvant sur la position 1 de la séquence d'acides aminés, codée par le codon d'initiation AUG ou GUG, et Xaa pouvant être un acide aminé quelconque,
et qui pour l'essentiel a une longueur de 220 acides aminés,
et
b) le gène cynT code pour un polypeptide dont la séquence d'acides aminés contient, entre les positions 50 et 71 ou des positions comparables, la séquence ThrGlnXaaGluProGlyXaaLeuPheValIleArgAsnAlaGlyAsnIleValP roSerXaaGly (SEQ ID N° 18), une méthionine se trouvant sur la position 1 de la séquence d'acides aminés, codée par le codon d'initiation AUG ou GUG, et Xaa pouvant être un acide aminé quelconque,
et qui pour l'essentiel a une longueur de 219 acides aminés,
les polypeptides possédant l'activité d'une carboanhydrase.

3. Micro-organismes selon la revendication 1, dans lesquels les polynucléotides désignés sous les noms de gène can et de gène cynT sont amplifiés à titre individuel ou en commun, le gène can et le gène cynT étant choisis dans le groupe consistant en :
a) le gène can, qui code pour un polypeptide dont la séquence d'acides aminés a une identité d'au moins 80 % avec une séquence d'acides aminés choisie dans le groupe SEQ ID N° 2, SEQ ID N° 4 et SEQ ID N° 6, et
b) le gène cynT, qui code pour un polypeptide dont la séquence d'acides aminés a une identité d'au moins 80 % avec une séquence d'acides aminés choisie dans le groupe SEQ ID N° 8, SEQ ID N° 10 et SEQ ID N° 12,
c) le gène can, qui code pour un polypeptide dont la séquence d'acides aminés a une identité d'au moins 95 % avec une séquence d'acides aminés choisie dans le groupe SEQ ID N° 2, SEQ ID N° 4 et SEQ ID N° 6, et
d) le gène cynT, qui code pour un polypeptide dont la séquence d'acides aminés a une identité d'au moins 95 % avec une séquence d'acides aminés choisie dans le groupe SEQ ID N° 8, SEQ ID N° 10 et SEQ ID N° 12, les polypeptides ayant l'activité d'une carboanhydrase,
e) le gène cynT ou can, qui codent pour des polypeptides dont les séquences d'acides aminés ont une identité de 100 % avec celles des séquences SEQ ID N° 2 ou SEQ ID N° 8.

4. Micro-organismes selon la revendication 3, **caractérisés en ce qu'**ils contiennent, amplifiés à titre individuel ou en commun, des polynucléotides isolés désignés sous le nom de gène can et de gène cynT, choisis dans le groupe :
a) le gène can, ayant une séquence nucléotidique choisie parmi SEQ ID N° 1, SEQ ID N° 3, SEQ ID N° 5, et le gène cynT, ayant une séquence nucléotidique choisie parmi SEQ ID N° 7, SEQ ID N° 9 et SEQ ID N° 11, et les séquences nucléotidiques qui leur sont complémentaires ;
b) le gène can, ayant une séquence nucléotidique choisie parmi les séquences qui correspondent à SEQ ID N° 1, SEQ ID N° 3 ou SEQ ID N° 5 dans le cadre de la dégénérescence du code génétique, et le gène cynT, ayant une séquence nucléotidique choisie parmi les séquences qui correspondent à SEQ ID N° 7, SEQ ID N° 9 ou SEQ ID N° 11 dans le cadre de la dégénérescence du code génétique ;
c) le gène can, ayant une séquence choisie parmi les séquences qui s'hybrident dans des conditions stringentes aux séquences complémentaires des séquences SEQ ID N° 1, SEQ ID N° 3 ou SEQ ID N° 5, et le gène cynT, ayant une séquence nucléotidique choisie parmi les séquences qui s'hybrident dans des conditions stringentes aux séquences complémentaires des séquences SEQ ID N° 7, SEQ ID N° 9, SEQ ID N° 11,
les conditions stringentes étant atteintes par une étape de lavage dans laquelle la température s'étend sur une plage de 64 à 68°C et la concentration du sel dans le tampon s'étend sur une plage de 2xSSC à 0,1×SSC ;
d) le gène can, ayant une séquence nucléotidique, pour le gène can, choisie parmi les séquences SEQ ID N° 1, SEQ ID N° 3 et SEQ ID N° 5, le gène cynT ayant une séquence nucléotidique choisie parmi les séquences SEQ ID N° 7, SEQ ID N° 9 et SEQ ID N° 11, qui contiennent les mutants sens fonctionnellement neutres,
les polynucléotides codant pour des carboanhydrases.

5. Micro-organismes selon les revendications 1 à 4, **caractérisés en ce qu'**ils sont préparés par transformation, par transduction, par conjugaison ou par une combinaison de ces procédés, avec un vecteur qui contient ledit gène can et/ou ledit gène cynT, un allèle de ces gènes, ou des parties de ces derniers et/ou un promoteur.

6. Micro-organismes selon les revendications 1 à 5, dans lesquels le nombre de copies du gène can et/ou du gène cynT mentionnés, ou des allèles, est augmenté d'au moins 1.

7. Micro-organismes selon la revendication 6, **caractérisés en ce que** l'augmentation, d'au moins 1, du nombre de copies du gène can et/ou du gène cynT mentionnés est réalisée par intégration du gène ou des allèles dans le chromosome des micro-organismes.

8. Micro-organismes selon la revendication 7, **caractérisés en ce que** l'augmentation, d'au moins 1, du nombre de copies du gène can et/ou du gène cynT mentionnés est atteinte par un vecteur à réplication extra-chromosomique.

9. Micro-organismes selon les revendications 1 à 8, **caractérisés en ce que**, pour réaliser l'amplification,
a) on mute la région promotrice et la région régulatrice, ou le site d'attachement du ribosome, en amont du gène can et/ou du gène cynT mentionnés, ou
b) on incorpore des cassettes d'expression ou des promoteurs en amont du gène can et/ou du gène cynT.

10. Micro-organismes selon les revendications 1 à 9, **caractérisés en ce que** l'expression du gène can et/ou du gène cynT mentionnés s'effectue sous le contrôle d'un promoteur amplifiant l'expression du gène.

11. Micro-organismes selon les revendications 1 à 10, **caractérisés en ce que**, sous l'effet de l'amplification du gène can et/ou du gène cynT mentionnés, les concentrations ou les activités des produits géniques codés (protéines) augmentent d'au moins 10 %, par rapport à l'activité ou à la concentration du produit génique dans le micro-organisme ou la souche parente non recombinant pour le gène can et/ou le gène cynT.

12. Micro-organismes selon les revendications 1 à 11, **caractérisés en ce que** les micro-organismes sont choisis parmi les genres Escherichia, Erwinia, Providencia et Serratia.

13. Procédé de préparation d'acides L-aminés par fermentation de micro-organismes recombinants de la famille des Enterobacteriaceae, **caractérisé en ce que**
a) on cultive les micro-organismes produisant l'acide L-aminé souhaité selon les revendications 1 à 12 dans un milieu dans des conditions dans lesquelles l'acide L-aminé souhaité va subir un enrichissement dans le milieu ou dans les cellules, et
b) on isole l'acide L-aminé souhaité, des constituants du bouillon de fermentation et/ou la biomasse restant en totalité ou en partie (≥ 0 à 100 %) dans le produit isolé, ou étant entièrement éliminés.

14. Procédé selon la revendication 13, **caractérisé en ce que**, pour la préparation de L-thréonine, on fermente des micro-organismes dans lesquels on affaiblit, en particulier on désactive, ou on en diminue l'expression, en plus et simultanément un ou plusieurs des gènes choisis dans le groupe consistant en :
a) le gène tdh codant pour la thréonine-déshydrogénase,
b) le gène mdh codant pour la malate-déshydrogénase,
c) le gène pckA codant pour la phosphoénolpyruvate-carboxykinase,
d) le gène poxB codant pour la pyruvate-oxydase,
e) le gène dgsA codant pour le régulateur DgsA du système phosphotransférase,
f) le gène fruR codant pour le répresseur fructose,
g) le gène rpoS codant pour le facteur Sigma³⁸, et
h) le gène aspA codant pour l'aspartate ammonium-lyase.

15. Procédé selon la revendication 13, **caractérisé en ce que**, pour préparer le L-trytophane, on fermente des micro-organismes de la famille des Enterobacteriaceae, dans lesquels on affaiblit, en particulier on désactive, ou on en diminue l'expression, en plus et simultanément un ou plusieurs des gènes choisis dans le groupe consistant en :
a) le gène tnaA codant pour la tryptophanase,
b) le gène trpR codant pour le répresseur de l'opéron trp,
c) le gène tyrA codant pour la chorismate-mutase T et la préphénate-déshydrogénase,
d) le gène pheA codant pour la chorismate-mutase P et la préphénate-déshydrogénase,
e) le gène mtr codant pour la protéine transporteuse spécifique du tryptophane,
f) le gène tnaB codant pour la tryptophane-perméase,
g) le gène aroP codant pour le transporteur d'acides aminés aromatiques,
h) le gène sdaA codant pour la L-sérine désaminase,
i) le gène pgi codant pour la glucose-6-phosphate-isomérase, et
j) le gène tyrB codant pour la tyrosine-aminotransférase.

16. Procédé selon la revendication 13, **caractérisé en ce qu'**on prépare des acides L-aminés choisis parmi la L-thréonine et le L-tryptophane.

17. Procédé selon les revendications 13 - 16, **caractérisé en ce qu'**on détermine la concentration de l'acide L-aminé à un ou plusieurs instants au cours de la fermentation.
